# EUROPEAN PATENT SPECIFICATION

(11) **EP 1 539 784 B1**
(45) Date of publication and mention of the grant of the patent: **28.10.2009**
(21) Application number: 02766120.6
(22) Date of filing: 26.08.2002
(51) Int. Cl.: C12N 15/56, C12N 15/82, C12N 9/24, C12N 5/10

(54) **CHITINASE ENCODING DNA MOLECULE FROM COTTON EXPRESSED PREFERENTIALLY IN FIBERS DURING SECONDARY CELL WALL DEPOSITION AND THE CORRESPONDING PROMOTER**
FÜR CHITINASE KODIERENDES DNA-MOLEKÜL AUS BAUMWOLLE, DAS VORZUGSWEISE IN FASERN WÄHREND DER BILDUNG DER ZWEITEN ZELLWAND EXPRIMIERT WIRD, UND DER ENTSPRECHENDE PROMOTOR
CHITINASE CODANT LA MOLECULE D'ADN DU COTON, S'EXPRIMANT DE FACON PREFERENTIELLE DANS LES FIBRES PENDANT LE DEPOT SECONDAIRE DANS LES PAROIS CELLULAIRES, ET PROMOTEUR CORRESPONDANT

(43) Date of publication of application: 15.06.2005
(73) Proprietor: TEXAS TECH UNIVERSITY, Lubbock, TX 79409-2007 (US)
(72) Inventor: HAIGLER, Candace, H., Lubbock, TX 79410 (US); ZHANG, Hong, Lubbock, TX 79414 (US); WU, Chunfa, Lubbock, TX 78413 (US); WAN, Chu-Hua, Medford, MA 02155 (US)
(74) Representative: Vaillant, Jeanne
(86) International application number: PCT/US2002/027227
(87) International publication number: WO 2004/018620

(56) References cited:
- ZHANG DESHUI ET AL: "Members of a new group of chitinase-like genes are expressed preferentially in cotton cells with secondary walls" PLANT MOLECULAR BIOLOGY, vol. 54, no. 3, February 2004 (2004-02), pages 353-372, XP002372436 ISSN: 0167-4412
- DATABASE UniProt [Online] 1 October 2000 (2000-10-01), "Basic chitinase (AT3g16920/K14A17_4)." XP002372440 retrieved from EBI accession no. UNIPROT:Q9LSP9 Database accession no. Q9LSP9
- HUDSPETH RICHARD L ET AL: "Characterization and expression of chitinase and 1,3-beta-glucanase genes in cotton" PLANT MOLECULAR BIOLOGY, vol. 31, no. 4, 1996, pages 911-916, XP009062134 ISSN: 0167-4412
- RINEHART ET AL.: 'Tissue-specific and developmental regulation of cotton gene FbL2A' PLANT PHYSIOL. vol. 112, 1996, pages 1331 - 1341, XP000973717
- SUNILKUMAR ET AL.: 'Cotton alpha-globulin promoter: isolation and functional characterization in transgenic cotton, Arabidiopsis, and tobacco' TRANSGENIC RES. vol. 11, August 2002, pages 347 - 359, XP009012418
- TAN ET AL.: 'Cloning and expression analysis of two cotton (Gossypium Hirshutum L.) genes encoding cell wall proline-rich proteins' DNA SEQUENCE vol. 12, 2001, pages 367 - 380, XP008040233
- ZHAO ET AL.: 'Isolation of a cotton RGP gene: a homolog reversibly glycosylated polypeptide highly expressed during fiber development' BIOCHIMICA ET BIOPHYSICA ACTA vol. 1574, 2001, pages 370 - 374, XP004353570
- CHLAN ET AL.: 'Class I chitinases in cotton (Gossypium hirsutum): characterization, expression and purification' PLANT SCIENCE vol. 161, 2001, pages 143 - 154, XP002903985
- JOHN ET AL.: 'Characterization of mRNA for a proline-rich protein of cotton fiber' PLANT PHYSIOL. vol. 108, 1995, pages 669 - 676, XP002903986

## Description

### FIELD OF THE INVENTION

The present invention relates to a gene that is expressed in fibers during secondary cell wall deposition and the promoter of the corresponding gene.

### BACKGROUND OF THE INVENTION

Fiber cells of cotton (*Gossypium hirsutum* L. and other *Gossypium* species including *G*. *barbadense* L., *G. arboreum* L., and *G*. *herbaceous* L.), a crop of enormous economic importance to world-wide agriculture, are differentiated epidermal cells of the seed coat. At maturity the fiber cell, considered from inside to outside, consists of a cell lumen, secondary cell wall, primary cell wall, and thin waxy cuticle. The primary cell wall is made up of pectic compounds, hemicellulose components, cellulose, and protein. The secondary cell wall consists mainly (about 95%) of cellulose with small percentages of other components not yet conclusively identified.

Cotton fiber development is characterized by the stages of initiation, primary cell wall deposition, secondary cell wall deposition, and dessication. During the primary wall stage of fiber development, primary wall deposition occurs to facilitate fiber elongation. During the secondary wall stage of fiber development, secondary wall deposition occurs to accomplish fiber thickening. Primary and secondary wall deposition involve the synthesis of all the cell wall components characteristic of each stage and the assembly of the molecules into an organized cell wall outside the plasma membrane. Many hundred of genes are required for the differentiation and development of plant fiber. Work on *in vitro* translated fiber proteins (Delmer et al., "New Approaches to the Study of Cellulose Biosynthesis," J. Cell Sci. Suppl., 2:33-50 (1985)), protein isolated from fiber (Graves and Stewart, "Analysis of the Protein Constituency of Developing Cotton Fibers," J. Exp. Bot., 39:59-69 (1988)), and analysis of particular genes (Wilkins et al., "Molecular Genetics of Developing Cotton Fibers," in Basra, ed., Cotton Fibers: Developmental Biology. Quality Improvement, and Textile Processing, Haworth Press:New York, p. 231-270 (1999)) clearly suggests differential gene expression during various developmental stages of the cell. However, only a few of the genes involved in the biosynthesis of the large numbers of fiber-specific or fiber-enhanced structural proteins, enzymes, polysaccharides, or waxes have been identified (John et al., "Gene Expression in Cotton (Gossypium hirsutum L.) Fiber: Cloning of the mRNAs," Proc. Natl. Acad. Sci. USA, 89:5769-5773 (1992); John, "Characterization of a Cotton (Gossypium hirsutum L.) Fiber mRNA (Fb-B6)," Plant Physiol., 107:1477-1478 (1995)). Since these genes and their interactions with environment determine the quality of fiber, their identification and characterization is considered to be an important aspect of cotton crop improvement.

In particular, how secondary cell walls are synthesized, how they aid plant function, adaptation, and defense, and how their properties translate into industrial utility are important questions related to basic biological mechanisms, ecology, and plant improvement. Plant secondary cell walls are synthesized in some specialized cell types to facilitate particular functions, such as long-range conduction of water (tracheary elements), control of transpiration (guard cells), and dispersal of seeds (cotton fibers). These secondary cell walls have a higher content of high tensile strength cellulose, usually exceeding 40% by weight, and are much thicker than primary cell walls. Consequently, their hemicellulose, pectin, and protein content is reduced, with the most extreme reduction occurring in the case of cotton fiber secondary cell walls which are about 95% cellulose. On the other hand, during primary wall deposition, the cellulose content is typically 9-30% (w/w) (Meinert et al., "Changes in Biochemical Composition of the Cell Wall of the Cotton Fiber During Development," Plant Physiol., 59:1088-1097 (1977); Darvill et al., "The Primary Cell Walls of Flowering Plants," The Biochemistry of Plants, 1:91-162 (1980); Smook, Handbook for Pulp and Paper Technologists, Vancouver, Canada:Angus Wilde Publications, p. 15 (1992)). Because secondary cell walls are strong and represent a bulk source of chemical cellulose, they have been exploited as important renewable resources, for example in wood and cotton fibers.

Cotton fiber cells have two distinct developmental stages that include secondary wall deposition. Many studies of fiber length and weight increase, morphology, cell wall composition, cellulose synthesis rates, and gene expression have confirmed the summary of the stages of fiber development presented below, and recent reviews contain many primary references to confirm these facts (Delmer, "Cellulose Biosynthesis in Developing Cotton Fibers," in Basra, ed., Cotton Fibers: Developmental Biology, Quality Improvement, and Textile Processing," New York, New York:Haworth Press, pp. 85-112 (1999); Ryser, "Cotton Fiber Initiation and Histodifferentiation," in Basra ed., Cotton Fibers: Developmental Biology, Quality Improvement, and Textile Processing," New York, New York:Haworth Press, pp. 1-46 (1999); Wilkins et al., "Molecular Genetics of Developing Cotton Fibers, in Basra, ed., Cotton Fibers: Developmental Biology Quality Improvement, and Textile Processing," New York, New York:Haworth Press, pp. 231-270 (1999)). Following fiber initiation by bulging of an epidermal cell above the surface, fiber elongation begins. Primary wall deposition is required to facilitate fiber elongation, and primary wall deposition continues alone for at least 12 days after fiber initiation. This period represents exclusively the primary wall stage of fiber development. Then secondary wall deposition begins while primary wall deposition continues, albeit usually at a slower rate. This stage of fiber development represents the transition between primary and secondary wall deposition, and it typically begins in *G*. *hirsutum* L. between 14 - 17 days post anthesis (DPA). Subsequently, fiber elongation and primary wall deposition cease, typically between 18 - 24 DPA, and secondary wall deposition persists exclusively until 34 - 50 DPA. Variation in the time of initiation and duration of each phase of fiber development depends on the cotton cultivar and the temperature conditions (DeLanghe, "Lint Development" in Mauney, eds., Cotton Physiology, Memphis, Tennessee:The Cotton Foundation, pp. 325-349 (1986); Haigler et al., "Cultured Cotton Ovules as Models for Cotton Fiber Development Under Low Temperatures," Plant Physiol., 95:88-96 (1991); Thaker et al., "Genotypic Variation and Influence of Diurnal Temperature on Cotton Fiber Development," Field Crops Research, 22:129-141 (1989)). For example, in field-grown *G*. *barbadense* L., extensive secondary wall deposition did not occur until after 20 DPA, elongation continued until 39 DPA, and secondary wall deposition ceased at 48 DPA (Schubert et al., "Growth and Development of the Lint Fibers of Pima S-4 Cotton," Crop Sci., 16:539-543 (1976)).

The rates of cellulose synthesis change from low, to medium, to high, respectively, at the primary wall, transition, and secondary wall stages of fiber development (Meinert et al., "Changes in Biochemical Composition of the Cell Wall of the Cotton Fiber During Development," Plant Physiol., 59:1088-1097 (1977); Martin, "Cool-Temperature-Induced Changes in Metabolism Related to Cellulose Synthesis in Cotton Fibers, Ph.D. dissertation, Texas Tech University, Lubbock, TX, U.S.A. (1999)). An example is found in fibers developing on cultured cotton ovules at an optimum temperature of constant 34°C, which maximizes the rate of progression through the stages of fiber development. Combining results from fibers on ovules of two cotton cultivars of *G*. *hirsutum L.* cultured under this condition, primary wall deposition along with a low rate of cellulose synthesis occurs until 12 - 14 DPA, the transition between primary and secondary wall deposition and an intermediate rate of cellulose synthesis begins at 14 - 16 DPA, and secondary wall deposition continues along with a high rate of cellulose synthesis beginning at 16 - 21 DPA (Martin, "Cool-Temperature-Induced Changes in Metabolism Related to Cellulose Synthesis in Cotton Fibers, Ph.D. dissertation, Texas Tech University, Lubbock, TX, U.S.A. (1999)). Other biochemical features demonstrate that the initiation of secondary wall deposition via an intermediate rate of cellulose synthesis at the transition stage marks a distinct developmental event: the cellulose content of new wall material rises sharply so that the overall percentage of cellulose in the whole fiber wall doubles in one day (Meinert et al., "Changes in Biochemical Composition of the Cell Wall of the Cotton Fiber During Development," Plant Physiol., 59:1088-1097 (1977)), the respiration rate transiently declines, and the intercellular pools in the fiber of UDP-glucose and glucose-6-P begin to rise (Martin, "Cool-Temperature-Induced Changes in Metabolism Related to Cellulose Synthesis in Cotton Fibers, Ph.D. dissertation, Texas Tech University, Lubbock, TX, U.S.A. (1999)). These are signs of an abrupt onset of secondary wall deposition (DeLanghe, "Lint Development" in Mauney, eds., Cotton Physiology, Memphis, Tennessee:The Cotton Foundation, pp. 325-349 (1986)).

Primary and secondary wall deposition appear to be controlled by different genetic factors (Kohel et al., "Fiber Elongation and Dry Weight Changes in Mutant Lines of Cotton," Crop Sci., 14:471-474 (1974)), and both stages can likely be manipulated independently by genetic engineering to achieve the longer, stronger, finer (smaller diameter), and more mature (as related to the secondary wall thickness) fiber that the textile industry desires. However, this goal can only be achieved by knowing more about the genes that control and contribute to each stage of fiber development.

After the fibers mature, the protective boll opens and the dried fiber often hangs for several weeks on the plant until the whole crop matures (unless stopped by killing cold temperatures) and vegetative growth dies or is killed chemically to allow harvest. During this period, fibers are subject to degradation by enzymatic activity of fungi, which is often enhanced by wet fall weather (Simpson et al., "The Geographical Distribution of Certain Pre-Harvest Microbial Infections of Cotton Fiber in the U.S. Cotton Belt," Plant Disease Reporter, 55:714-718 (1971)). In some years, this field-waiting time causes substantial deterioration of the grade of the fiber so that the producer receives a discounted price and the production of quality yarns and fabrics is jeopardized. Therefore, cotton production efficiency will be improved by more knowledge of how to bring the fibers undamaged from the field to textile plant. Relevant to achieving this goal is a better understanding of endogenous protections against fungal degradation that could be introduced or enhanced in the fiber.

Particularly because of their defensive role in plants (Gooday, "Aggressive and Defensive Roles for Chitinases," in Jolles, eds., Chitin and Chitinases, Birkhäuser Verlag:Basel, pp. 157-170 (1999)), numerous chitinase genes and proteins have been characterized in diverse plant species. The chitinase gene and protein family has been the subject of many reviews (including Graham et al., "Cellular Coordination of Molecular Responses in Plant Defense," Molecular Plant-Microbe Interactions: MPMI, 4:415-422 (1991); Cutt et al., "Pathogenesis-Related Proteins," in Boller, eds., Genes Involved in Plant Defense, Springer Verlag/New York. pp. 209-243 (1992); Meins et al., "The Primary Structure of Plant Pathogenesis-Related Glucanohydrolases and Their Genes," in Boller, eds., Genes Involved in Plant Defense Springer Verlag/New York, p. 245-282 (1992); Collinge et al., "Plant Chitinases," Plant J., 3:31- 40 (1993); Sahai et al., "Chitinases of Fungi and Plants: Their Involvement in Morphogenesis and Host-Parasite Interaction," FEMS Microbiology Rev., 11:317-338 (1993); Meins et al., "Plant Chitinase Genes," Plant Molecular Biology Reporter, 12:522-528 (1994); Hamel et al., "Structural and Evolutionary Relationships Among Chitinases of Flowering Plants," Journal of Molecular Evolution, 44:614-624 (1997)) and of an edited book (Jolles, eds. Chitin and Chitinases, Birkhäuser Verlag:Basel, 340 pp (1999)). These sources contain many primary references to the well known facts summarized below. Chitinases are among a group of genes that are inducible in plants by pathogen attack, corresponding to the frequent occurrence of chitin in fungal cell walls and insect exoskeletons. In their defensive role, chitinases catalyze the hydrolysis of chitin. Structural chitin occurs as crystalline microfibrils composed of a linear homopolymer of β-1,4-linked N-acetyl-D-glucosamine residues, (GlcNAc)ₙ. Chitin hydrolysis defends the plant against predators or pathogens, particularly invading fungi, by weakening or dissolving their body structure. Especially in combination with β-1,3-glucanases that serve to uncoat the chitin microfibrils, chitinases can inhibit the growth of many fungi by causing hyphal tip lysis due to a weakened hyphal wall. This has been shown by inhibition of fungal growth in cultures as well as in transgenic plants that exhibit reduced pathogen damage in correlation with increased chitinase activity. Gene expression or activity of chitinases with a probable defensive function have previously been characterized in cotton leaves and roots (Liu et al., "Detection of Pathogenesis-Related Proteins in Cotton Plants," Physiological and Molecular Plant Pathology, 47:357-363 (1995); Hudspeth et al., "Characterization and Expression of Chitinase and 1,3-β-Glucanase Genes in Cotton," Plant Molecular Biology, 31:911-916 (1996); Dubery et al., "Induced Defence Responses in Cotton Leaf Disks by Elicitors From Verticillium dahliae," Phytochemistry, 44: 1429-1434 (1997)).

Some chitinases are induced upon fungal invasion, accumulating around invading fungal hyphae (Benhamou et al., "Subcellular Localization of Chitinase and Its Potential Substrate in Tomato Root Tissues Infected With Fusarium Oxysporium F.Sp. Radicislycopersici," Plant Physiology, 92:1108-1120 (1990); Wubben et al., "Subcellular Localization of Plant Chitinases and 1,3-β-Glucanases in Cladosporium Fulvum (Syn. Fulvia Fulva) Infected Tomato Leaves," Physiological and Molecular Plant Pathology 41:23 - 32 (1992)). Other chitinases apparently occur constitutively in plant parts that are particularly susceptible to invasion such as epidermal cells, root cortical cells, stomates, flower parts, and vascular cells. These conclusions arise from both localization of chitinase mRNA by *in situ* hybridization and analysis of patterns of expression of the GUS reporter gene under control of chitinase promoters (Samac et al., "Developmental and Pathogen-Induced Activation of the Arabidopsis Acidic Chitinase Promoter," The Plant Cell, 3:1063-1072 (1991); Zhu et al., "Stress Induction and Developmental Regulation of a Rice Chitinase Promoter in Transgenic Tobacco," The Plant Journal, 3:203-212 (1993); Büchter et al., "Primary Structure and Expression of Acidic (Class II) Chitinase in Potato," Plant Molecular Biology, 35:749-761 (1997); Ancillo et al., "A Distinct Member of the Basic (Class I) Chitinase Gene Family in Potato is Specifically Expressed in Epidermal Cells,". Plant Molecular Biology, 39:1137-1151 (1999)). In another case of possible anticipation of fungal invasion in disrupted tissues, ethylene induces chitinase in bean abscission zones (del Campillo et al., "Identification and Kinetics of Accumulation of Proteins Induced by Ethylene in Bean Abscission Zones," Plant Physiology, 98:955-961 (1991)). None of these studies included analysis of cotton fibers or showed the presence of chitinase activity or chitinase-related proteins in cotton fibers.

Other studies show that some chitinases have a developmental role in plants, although authentic structural chitin is not a natural part of the plant body (Meins et al., "The Primary Structure of Plant Pathogenesis-Related Glucanohydrolases and Their Genes," In Boller, eds., Genes Involved in Plant Defense, Springer Verlag/New York, p. 245-282 (1992)). It has been shown that chitinase isoforms with developmental roles are at least sometimes distinct from those with roles in stress responses and defense (Mauch et al., "Antifungal Hydrolases in Pea Tissue. I. Purification and Characterization of Two Chitinases and Two β-1,3-Glucanases Differentially Regulated During Development and in Response to Fungal Infection," Plant Physiology, 87:325-333 (1988)). Defensive chitinases bind to short stretches (probably 3-6 residues) of a single N-acetyl-glucosamine chain prior to cleaving the inter-sugar bond (Robertus et al., "The Structure and Action of Chitinases," in Jolles, eds., Chitin and Chitinases, Birkhäuser Verlag:Basel, pp. 125-136 (1999)). Therefore, enzymes in the chitinase family can also bind to oligomers ofN-acetyl-glucosamine within other molecules such as glycoproteins or signalling molecules. Such molecules may have roles in signal transduction to regulate gene expression cascades required for developmental transitions or in the biosynthetic processes that implement the developmental program.

Previous research on the regulation of secondary wall deposition or function at the molecular level focused on a few genes involved in cellulose, hemicellulose, lignin, or protein biosynthesis. Among these pathways, lignin synthesis has been most fully explored and manipulated in transgenic plants (Merkle et al., "Forest Biotechnology," Current Opinion in Biotechnology, 11:298-302 (2000)). However, cotton fibers contain no lignin (Haigler, "The Functions and Biogenesis of Native Cellulose," in Zeronian, eds., Cellulose Chemistry and Its Applications, Ellis Horwood:Chichester, England, pp. 30-83 (1985)). Hemicellulose polysaccharides within some secondary walls include xylans and glucomannans, but cotton fiber secondary walls do not contain significant quantities of any similar molecule (Haigler, "The Functions and biogenesis of Native Cellulose," in Zeronian, eds., Cellulose Chemistry and Its Applications, Ellis Horwood:Chichester, England, pp. 30-83 (1985)). Only two proteins with possible structural roles in the cotton fiber secondary wall have been identified. One of these, H6, is an arabinogalactan-type protein that accumulates to detectable levels during secondary wall deposition although the expression of its gene begins during rapid elongation including primary wall deposition (John et al., "Characterization of mRNA for a Proline-Rich Protein of Cotton Fiber," Plant Physiology, 108:669-676 (1995)). The second, FbL2A, lacks homology to any known protein, but its highly repetitive sequence and high hydrophilicity suggest that it may have a structural role or protect cotton fibers during dessication. The expression of its gene begins weakly at the primary to secondary wall transition (15 DPA) and is stronger by 20 DPA (Rinehart et al., "Tissue-Specific and Developmental Regulation of Cotton Gene FbL2A," Plant Physiology, 112:1331-1341 (1996)).

Enzymes that increase in gene expression and/or activity during cotton fiber secondary wall deposition and that relate to the regulation of cellulose synthesis include cellulose synthase, sucrose synthase, sucrose phosphate synthase, and UDP-glucose pyrophosphorylase (Basra et al., " Sucrose Hydrolysis in Relation to Development of Cotton (Gossypium spp.) Fibres," Indian Journal of Experimental Botany, 28:985-988 (1990); Wäfler et al., "Enzyme Activities in Developing Cotton Fibres," Plant Physiology and Biochemistry, 32:697-702 (1994); Amor et al., "A Membrane-Associated Form of Sucrose Synthase and its Potential Role in Synthesis of Cellulose and Callose in Plants," Proc. Nat'l. Acad. Sci. U.S.A., 92:9353-9357 (1995); Pear et al., "Higher Plants Contain Homologs of the Bacterial celA Genes Encoding the Catalytic Subunit of Cellulose Synthase," Proc. Nat'l. Acad. Sci. U.S.A., 93:12637-12642 (1996); Tummala, "Response of Sucrose Phosphate Synthase Activity to Cool Temperatures in Cotton," M.S. thesis, Texas Tech University, Lubbock, TX (1996)). UDP-glucose pyrophosphorylase converts glucose-1-P to UDP-glucose or mediates the reverse reaction. In the context of cellulose synthesis, sucrose synthase is thought to degrade sucrose and supply UDP-glucose to cellulose synthase. Cellulose synthase transfers the glucose to the elongating β-1,4-linked cellulose polymer while free UDP is recycled to sucrose synthase. Sucrose phosphate synthase may use fructose-6-P (e.g. that derived from the fructose released by the degradative action of sucrose synthase) and UDP-glucose to synthesize additional sucrose to support cellulose synthesis (Delmer, "Cellulose Biosynthesis in Developing Cotton Fibers," in Basra, ed., Cotton Fibers: Developmental Biology, Quality Improvement, and Textile Processing, Haworth Press:New York, pp. 85 - 112 (1999)).

All of the enzymes just discussed operate within the pathways of sugar metabolism leading to the formation of the β-1,4-linked glucan polymer. Evidence from other systems and cotton fibers implicates other possible points of regulation of cellulose synthesis coincident with or after formation of the glucan polymer, although the relevant pathways and proteins are incompletely understood. Examples of other relevant proteins include β-1,4-glucanase that may act as a glucan chain editor or in some other role (Delmer, " Cellulose Biosynthesis: Exciting Times For a Difficult Field of Study," Ann. Rev. Plant Physiol. Mol. Biol., 50:245-276 (1999 b)) and glycoproteins that may act as primers for cellulose biosynthesis (Lukowitz et al., "Arabidopsis cyt1 Mutants are Deficient in a Mannose-1-Phosphate Guanyltransferase and Point to a Requirement ofN-Linked Glycosylation for Cellulose Biosynthesis," Proc. Nat'l. Acad. Sci. U.S.A., 98:2262-2267 (2001)). Mutations that down-regulate β-1,4-glucanase or glycoprotein synthesis cause reduced cellulose content in other systems (Nicol et al., " Plant Cell Expansion: Scaling the Wall," Current Opinion in Cell Biology, 1:12-17 (1998); Lukowitz et al., "Arabidopsis cyt1 Mutants are Deficient in a Mannose-1-Phosphate Guanyltransferase and Point to a Requirement of N-Linked Glycosylation for Cellulose Biosynthesis," Proc. Nat'l. Acad. Sci. U.S.A., 98:2262-2267 (2001)). In *Arabidopsis*, mutation of xylem secondary wall specific cellulose synthase genes causes reduced cellulose content and weak xylem walls (Turner et al., "Collapsed Xylem Phenotype of Arabidopsis Identifies Mutants Deficient in Cellulose Deposition in the Secondary Cell Wall," Plant Cell, 9:689 - 701 (1997)). In cotton, increased expression of spinach sucrose phosphate synthase causes increased cellulose content in fiber walls of plants growing under a cool night cycle (Haigler et al., "Transgenic Cotton Over-Expressing Sucrose Phosphate Synthase Produces Higher Quality Fibers With Increased Cellulose Content and Has Enhanced Seed Cotton Yield," Abstract 477. In: Proceedings of Plant Biology 2000, July 15 - 19, San Diego, CA. American Society of Plant Physiologists, Rockville, MD, (2000)). These findings indicate that it is possible to manipulate the quantity of cellulose in secondary walls, but presently there is insufficient identification of target genes that might be beneficially manipulated.

Studies that identify genes that are under tissue-specific and developmental regulation are important in understanding the roles of proteins in fiber development and cell-wall architecture (John, "Structural Characterization of Genes Corresponding to Cotton Fiber mRNA, E6: Reduced E6 Protein in Transgenic Plants by Antisense Gene," Plant Mol. Biol., 30:297-306 (1996)). In addition, such genes and their regulatory elements are important tools for fiber modification through genetic engineering (John, "Prospects for Modification of Fibers Through Genetic Engineering of Cotton," in Gebelein, eds., Industrial Biotechnological Polymers, Lancaster, PA:Technomic, pp. 69-79 (1995); John, "Genetic Engineering Strategies for Cotton Fiber Modification. In: A.S. Basra (ed.), Cotton Fibers: Developmental Biology, Quality Improvement, and Textile Processing, The Haworth Press, New York, pp. 271- 289 (1999)).

In many instances, it would be desirable for a transgene to be developmentally regulated to have exclusive or preferential expression in fiber cells at a proper developmental stage. This regulation can be most expeditiously accomplished by a promoter capable of preferential promotion.

Promoters are DNA elements that direct the transcription of RNA in cells. Together with other regulatory elements that specify tissue and temporal specificity of gene expression, promoters control the development of organisms. Thus, there has been a concerted effort in identifying and isolating promoters from a wide variety of plants and animals.

Many promoters function properly in heterologous systems. For example, promoters taken from plant genes such as rbcS, Cab, chalcone synthase, and protease inhibitor from tobacco and Arabidopsis are functional in heterologous transgenic plants. (Benfey et al., "Regulated Genes in Transgenic Plants," Science, 244:174-181, (1989)). Specific examples of transgenic plants include tissue-specific and developmentally regulated expression of soybean 7s seed storage protein gene in transgenic tobacco plants (Chen et al., "A DNA Sequence Element That Confers Seed-Specific Enhancement to a Constitutive Promoter," EMBO J., 7:297-302, (1988)) and light-dependent organ-specific expression of *Arabidopsis thaliana* chlorophyll a/b binding protein gene promoter in transgenic tobacco (Ha et al., "Identification of Upstream Regulatory Elements Involved in the Developmental Expression of the Arabidopsis-thaliana Cab-1 Gene," Proc. Natl. Acad. Sci. USA, 85:8017-8021, (1988)). Similarly, anaerobically inducible maize sucrose synthase-1 promoter activity was demonstrated in transgenic tobacco (Yang et al., "Maize Sucrose Synthase-1 Promoter Directs Phloem Cell-Specific Expression of Gus Gene in Transgenic Tobacco Plants," Proc. Natl. Acad. Sci. USA, 87: 4144-4148, (1990)). Tomato pollen promoters were found to direct tissue-specific and developmentally regulated gene expression in transgenic Arabidopsis and tobacco (Twell et al., "Pollen-Specific Gene Expression in Transgenic Plants Coordinate Regulation of Two Different Tomato Gene Promoters During Microsporogenesis," Development, 109:705-714, 1990). Thus, some plant promoters can be utilized to express foreign proteins in plant tissues in a developmentally regulated fashion.

Tissue-specific and developmentally regulated expression of genes has also been shown in fiber cells. Several of these, such as E6, vacuolar ATPase, and lipid transfer-type proteins, have strong expression in cotton fibers during primary wall deposition (Wilkins et al., "Molecular Genetics of Developing Cotton Fibers," in Basra, ed., Cotton Fibers: Developmental Biology, Quality Improvement, and Textile Processing, Haworth Press:New York, p. 231-270 (1999); Orford et al., "Expression of a Lipid Transfer Protein Gene Family During Cotton Fibre Development," Biochimica and Biophysica Acta, 1483:275-284 (2000)). Other genes show transient expression during fiber development. For example, Rac is transiently expressed at the primary to secondary wall stage transition (Delmer et al., "Genes Encoding Small GTP-Binding Proteins Analogous to Mammalian Rac are Preferentially Expressed in Developing Cotton Fibers," Mol. Gen. Genet., 248:43-51 (1995)) and another lipid transfer-type protein, FS18A (Orford et al., " Characterization of a Cotton Gene Expressed Late in Fibre Cell Elongation," Theoretical and Applied Genetics, 98:757-764 (1999)), is transiently expressed at 24 DPA during secondary wall deposition. Another gene, H6, is expressed between 10 - 24 DPA, which includes both primary and early secondary wall deposition, but H6 protein accumulates only during secondary wall deposition at 15 - 40 DPA (John et al., " Characterization of mRNA for a Proline-Rich Protein of Cotton Fiber," Plant Physiology, 108:669-676 (1995)). At the level of gene expression, only certain cellulose synthase genes have been previously shown to have preferential and prolonged expression in cotton fibers during secondary wall deposition, although there were lower levels of expression during primary wall deposition and in other parts of the plant. In addition, cellulose synthase did not show strong expression until 20 DPA, with only weak expression observed at 17 DPA (Pear et al., "Higher Plants Contain Homologs of the Bacterial celA Genes Encoding the Catalytic Subunit of Cellulose Synthase," Proc. Nat'l. Acad. Sci. U.S.A., 93:12637-12642 (1996)). Another gene, FbL2A, is up-regulated at the primary to secondary wall transition (weakly at 15 DPA and strongly at 20 DPA), but data on gene expression during later stages of fiber development were not shown (Rinehart et al., "Tissue-Specific and Developmental Regulation of Cotton Gene FbL2A," Plant Physiology, 112:1331-1341 (1996)).

It would be useful to have a promoter that would drive gene expression preferentially and strongly in fibers throughout secondary wall deposition, i.e., strongly and continuously (e.g. at ≥ 50% of its maximal activity) from the initiation of secondary wall deposition to its termination. The initiation of secondary wall deposition is defined as the time when the dry weight/unit length of a cotton fiber begins to increase or when the dry weight/unit surface area of any cell begins to increase via synthesis of new wall material containing more than 40% (w/w) of cellulose. In the case of cotton fiber of *G*. *hirsutum* L., this is expected to occur between 14 - 17 DPA when cotton plants are grown under typical conditions in the greenhouse or the field (day temperature of 26 - 34°C, night temperature of 20 - 26°C, light intensity greater than or equal to 1000 µeinsteins/m²/s, with adequate water and mineral nutrition). Furthermore, it would be useful to have a promoter that would drive gene expression only or preferentially in fibers while excluding or minimizing expression in other tissues.

The present invention is directed to achieving these objectives.

### SUMMARY OF THE INVENTION

The present invention relates to an isolated nucleic acid molecule from cotton encoding an endogenous cotton protein related to chitinase, where the nucleic acid molecule is expressed preferentially in fibers during secondary wall deposition. A polypeptide encoded by the isolated nucleic acid molecule is also disclosed.

Another aspect of the present invention relates to a DNA construct comprising a promoter operably linked 5' to a nucleic acid molecule as described herein, and a 3' regulatory region operably linked to the nucleic acid molecule wherein said promoter is foreign to said nucleic acid molecule. The DNA construct can be incorporated in an expression system, a host cell, a plant, or a plant seed.

Also disclosed is a method of imparting resistance to plants against insects and fungi composing transforming a plant with the DNA construct comprising the isolated nucleic acid molecule of the present invention.

Another aspect of the present invention relates to a method of regulating the cellulose content of a plant fiber comprising transforming a plant with the DNA construct comprising the isolated nucleic acid molecule of the present invention.

The finding that the isolated nucleic acid molecule disclosed in the present invention is expressed preferentially in fibers during secondary wall deposition when cellular activity is strongly skewed toward cellulose synthesis is consistent with a developmental role for the corresponding chitinase-related protein in cellulose synthesis. Alternatively, the protein could be "stored" in the cotton fiber cell wall awaiting fungal attack after boll opening. However, any chitinase with this defensive function in cotton fibers may be relatively ineffective because cotton fibers can be infected and greatly damaged by several fungal species (Simpson et al., "The Geographical Distribution of Certain Pre-Harvest Microbial Infections of Cotton Fiber in the U.S. Cotton Belt," Plant Disease Reporter, 55:714-718 (1971)). Whether acting developmentally or defensively, manipulating the expression of these chitinase-related proteins in fibers could have important consequences for fiber crop production and quality. For example, fiber cellulose content could be enhanced or diminished during biosynthesis and/or protected from fungal degradation during crop harvesting and processing.

In addition, a promoter of a gene that is expressed preferentially in fibers during secondary wall deposition of normal plants can be valuable for genetic engineering of fiber to achieve: (1) improved agricultural productivity under normal and stressed conditions and (2) improved fiber properties that depend on modification during secondary wall deposition. Although the promoter for the FbL2A gene has been tested by fusion to two reporter genes (polyhydroxyalkanoic acid synthase or PHA synthase, an enzyme that was detected with a specific antibody, and acetoacetyl-CoA reductase, an enzyme with activity that was monitored by enzyme assay) and transformation of cotton (Rinehart et al., " Tissue-Specific and Developmental Regulation of Cotton Gene FbL2A," Plant Physiology, 112:1331-1341 (1996)), expression of the gene FbL2A was shown in cotton fibers weakly at 15 DPA and strongly at 20 DPA with no data provided for the duration of secondary wall deposition until 30 DPA or later. By use of the two promoter/reporter gene constructs, somewhat contradictory data were provided on the utility of the promoter of FbL2A. When the acetoacetyl-CoA reductase gene was under control of the FbL2A promoter, acetoacetyl-CoA reductase activity was detected in cotton fibers during primary wall deposition at 5 - 10 DPA, and there was consistent, substantial activity by 20 DPA during secondary wall deposition, peak activity at 35 DPA, and continued activity until 45 DPA among a family of independently transformed plants (Rinehart et al., "Tissue-Specific and Developmental Regulation of Cotton Gene FbL2A," Plant Physiology, 112:1331-1341 (1996)). However, the enzyme activity after 20 DPA could be due to long-lived messenger RNA or protein synthesized at 15 - 20 DPA, which is the period when the data directly show FbL2A gene expression under control of its own promoter. In contrast, when the PHA synthase gene was under the control of the FbL2A promoter, Western blotting to detect PHA synthase immunologically in a transformed plant showed only very weak signal during secondary wall deposition at 20 DPA, a trace signal at 25 DPA, and no signal at 30 or 35 DPA (Rinehart et al., "Tissue-Specific and Developmental Regulation of Cotton Gene FbL2A," Plant Physiology, 112:1331-1341 (1996)). Furthermore, the PHA synthase gene under the control of the putatively consitutive 35S promoter from CaMV virus correlated with detection of PHA synthase protein strongly during primary wall deposition at 10 DPA and weakly continuing through 35 DPA of secondary wall deposition. The comparative patterns during secondary wall deposition are consistent with transient expression of the FbL2A promoter around 20 DPA of secondary wall deposition. The data also show that the FbL2A promoter will drive weak foreign gene expression in cotton fibers during primary wall deposition (Rinehart et al., "Tissue-Specific and Developmental Regulation of Cotton Gene FbL2A," Plant Physiology, 112:1331-1341 (1996)). Therefore, there is no known gene that encodes a functional protein that is preferentially and strongly expressed in fibers throughout secondary wall deposition. Correspondingly, there is no promoter to drive foreign gene expression preferentially and strongly throughout the whole duration of fiber secondary wall deposition. The promoter disclosed in the present invention allows these goals to be met while avoiding or minimizing pleiotropic effects on plant growth and development or other stages of fiber development that could reduce the benefit of the targeted effects.

### BRIEF DESCRIPTION OF THE DRAWINGS

Figure 1 is an autoradiographic image on film of a gel of labeled cDNA fragments arising through the technique of differential display from RNA isolated from 18 and 12 DPA cotton fibers, respectively, of *Gossypium hirsutum* cv. Coker 312. The arrow points to the band unique to 18 DPA cotton fibers that corresponds to the gene subsequently named F285 and shown to have homology to chitinases.

Figure 2 shows a Northern blot exposed to film overnight showing total RNA isolated from various cotton tissues. The top frame shows incubation with a probe to F285, and the bottom frame shows incubation with a probe to 18S RNA to show the comparative level of RNA loading in each lane, which was intended to be 15 µg/lane.

Figure 3 shows a Northern blot, illustrating a more detailed time-course of fiber development. The top frame shows incubation with a probe to F285, and the bottom frame shows incubation with a probe to 18S RNA to show the comparative level of RNA loading in each lane.

Figure 4 illustrates the detection of chitinase activity in protein extracts of various tissues as follows:
Area 1: 8 DPA greenhouse-grown fibers, untreated before protein extraction
Area 2: 17 DPA greenhouse-grown fibers, untreated before protein extraction
Area 3: 24 greenhouse-grown fibers, untreated before protein extraction
Area 4: 31 greenhouse-grown fibers, untreated before protein extraction
Area 5: 18 day old leaves, untreated before protein extraction.
Area 6: 18 day old leaves, water-treated at 16 days after planting
Area 7: 18 day old leaves, salicylic acid (SA)-treated at 16 days after planting
Area 8: Negative control (Water)
Area 9: 8 DPA cultured fibers, water-treated
Area 10: 8 DPA cultured fibers, SA-treated on 6 DPA
Area 11: Negative control (BSA solution)
Area 12: Positive control (Commercial bacterial chitinase)

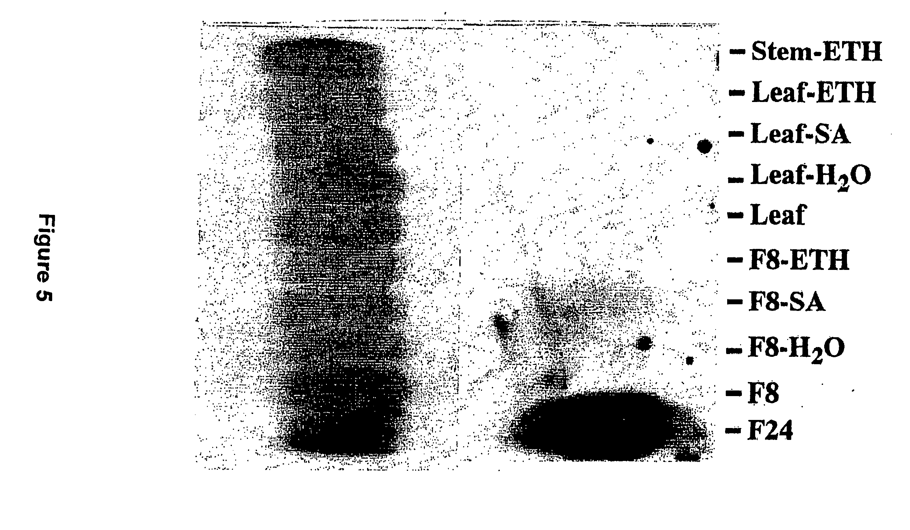

Figure 5 shows a Northern blot, testing for induction by SA or ethephon (ETH) of expression of F285. The top frame shows incubation with a probe to F285, and the bottom frame shows incubation with a probe to 18S RNA to show the comparative level of RNA loading in each lane. The tissue and pre-treatment, if any, are indicated above each lane. Treatments, if any, were applied to seedlings 16 days after planting, and organs were harvested two days later. Treatments, if any, were applied to ovules at 6 DPA with harvest occurring at 8 DPA.

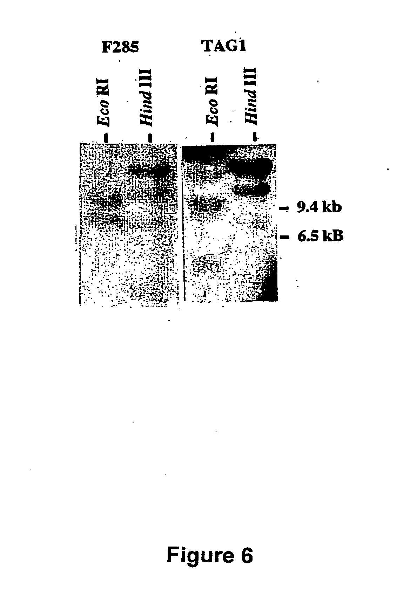

Figure 6 shows a Southern blot of cotton genomic DNA digested with EcoRI and Hind III restriction enzymes and probed with the F285 full-length cDNA and, subsequently, with a fragment of another member of its gene family, TAG1.

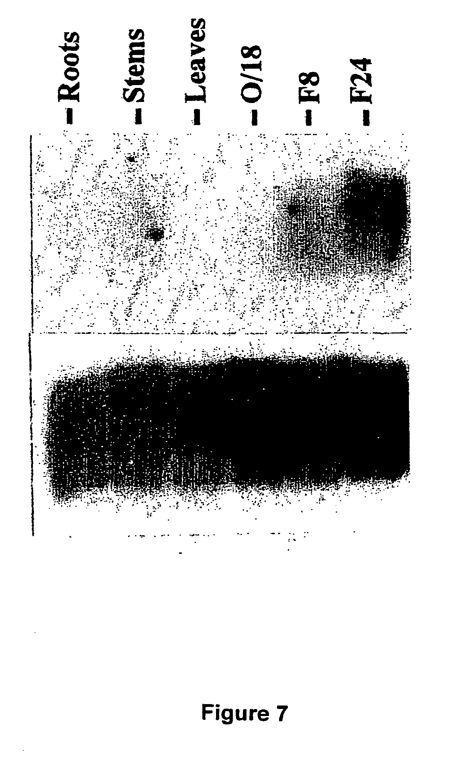

Figure 7 shows a Northern blot testing the expression of TAG 1. The top frame shows incubation with a probe to TAG1, and the bottom frame shows incubation with a probe to 18S RNA to show the comparative level of RNA loading in each lane. Tissues tested, including roots, stems, and leaves, and stripped ovules at 18 DPA, fibers at 8 DPA, and fibers at 24 DPA, are indicated above each lane.

### DETAILED DESCRIPTION OF THE INVENTION

The present invention relates to an isolated nucleic acid molecule from cotton encoding an endogenous cotton chitinase. The nucleic acid molecule is expressed preferentially in fibers during secondary wall deposition. Typically, the fiber is cotton fiber.

Preferential expression in fibers during secondary wall deposition means gene expression in the fiber cell during secondary wall deposition at a level more than 100 times higher than in other cell types or at other stages of cell development. Comparative levels of gene expression can be assessed by various standard molecular biological techniques including semi-quantitative Northern blotting and real-time quantitative PCR.

The word "fiber" is often used to unify a diverse group of plant cell types that share in common the features of having an elongated shape and abundant cellulose in thick cell walls, usually, but not always, described as secondary walls. Such walls may or may not be lignified, and the protoplast of such cells may or may not remain alive at maturity. Such fibers have many industrial uses, for example in lumber and manufactured wood products, paper, textiles, sacking and boxing material, cordage, brushes and brooms, filling and stuffing, caulking, reinforcement of other materials, and manufacture of cellulose derivatives. In some industries, the term "fiber" is usually inclusive of thick-walled conducting cells such as vessels and tracheids and to fibrillar aggregates of many individual fiber cells. Here the term "fiber" is used in its most inclusive sense, for example including: (a) thick-walled conducting and non-conducting cells of the xylem; (b) fibers of extraxylary origin, including those from phloem, bark, ground tissue, and epidermis; and (c) fibers from stems, leaves, roots, seeds, and flowers or inflorescences (such as those of *Sorghum vulgare* used in the manufacture of brushes and brooms). In addition to wood from trees, cotton, and forage crops, the invention is applicable to all fibers, including, but not exclusively, those in agricultural residues such as corn, sugar cane, and rice stems that can be used in pulping, flax, hemp, ramie, jute, kenaf, kapok, coir, bamboo, spanish moss, abaca, and *Agave* spp. (e.g. sisal).

The isolated nucleic acid molecule of the present invention can have a nucleotide sequence of SEQ. ID. No. 1 as follows:

The isolated nucleic acid molecule of the present invention also comprises a nucleotide sequence which hybridizes to a DNA molecule having a sequence according to SEQ. ID. No. 1 under stringent conditions characterized by a hybridization buffer comprising 1 x SSC at a temperature of 61°C.

The isolated nucleic acid molecule of SEQ. ID. No. 1 encodes a protein or polypeptide having a deduced amino acid sequence of SEQ. ID. No. 2 as follows:

This amino acid sequence shows homology to plant chitinases that are important in the defense or in the development of the cotton fiber. The present invention also relates to a polypeptide which is encoded by the isolated nucleic acid molecule of the present invention and has an amino acid sequence corresponding to SEQ. ID. No. 2.

The isolated nucleic acid molecule of the present invention is preferentially expressed in cotton fibers beginning at 14 to 17 DPA, preferably extending through to the termination of secondary wall deposition. Most preferably, the isolated nucleic acid molecule of the present invention is preferentially expressed in cotton fibers beginning at 14 to 17 DPA up to 31 DPA. The isolated nucleic acid molecule of the present invention is the first cotton gene with homology to chitinase that is known to be expressed preferentially in fibers during secondary wall deposition. The gene's precise temporal regulation with secondary wall deposition suggests that the gene could be manipulated to change fiber development or defensive properties.

In a preferred form of the present invention, the isolated nucleic acid molecule of the invention is in a DNA construct comprising a promoter operably linked 5' to a second DNA encoding a protein or polypeptide to induce transcription of the second DNA. The second DNA comprises the isolated nucleic acid molecule of the invention. A 3' regulatory region is operably linked to the nucleic acid molecule wherein said promoter is foreign to said nucleic acid molecule.

In another embodiment, the present invention is an expression system that includes a suitable vector containing the DNA construct of the invention. The expression system contains the necessary elements for the transcription and translation of the inserted protein-coding sequences. In preparing the DNA construct for expression, the various DNA sequences may normally be inserted or substituted into a bacterial plasmid. Any convenient plasmid may be employed, which will be characterized by having a bacterial replication system, a marker which allows for selection in a bacterium, and generally one or more unique, conveniently located restriction sites. Numerous plasmids, referred to as transformation vectors, are available for plant transformation. The selection of a vector will depend on the preferred transformation technique and target species for transformation. A variety of sectors are available for stable transformation using *Agrobacterium tumefaciens*, a soilborne bacterium that causes crown gall. Crown gall is characterized by tumors or galls that develop on the lower stem and main roots of the infected plant. These tumors are due to the transfer and incorporation of part of the bacterium plasmid DNA into the plant chromosomal DNA. This transfer DNA (T-DNA) is expressed along with the normal genes of the plant cell. The plasmid DNA, pTI, or Ti-DNA, for "tumor inducing plasmid," contains the *vir* genes necessary for movement of the T-DNA into the plant. The T-DNA carries genes that encode proteins involved in the biosynthesis of plant regulatory factors, and bacterial nutrients (opines). The T-DNA is delimited by two 25 bp imperfect direct repeat sequences called the "border sequences." By removing the oncogene and opine genes, and replacing them with a gene of interest, it is possible to transfer foreign DNA into the plant without the formation of tumors or the multiplication of *A*. *tumefaciens* (Fraley et al., "Expression of Bacterial Genes in Plant Cells," Proc. Nat'l Acad. Sci USA, 80: 4803-4807 (1983),

Further improvement of this technique led to the development of the binary vector system (Bevan, M., "Binary Agrobacterium Vectors for Plant Transformation," Nucleic Acids Res., 12:8711-8721 (1984) In this system, all the T-DNA sequences (including the borders) are removed from the pTi, and a second vector containing T-DNA is introduced into *A. tumefaciens*. This second vector has the advantage of being replicable in *E*. *coli* as well as *A. tumefaciens* and contains a multiple cloning site that facilitates the cloning of a transgene. An example of a commonly used vector is pBin19 (Frisch, et al., "Complete Sequence of the Binary Vector Bin19," Plant Molec. Biol., 27:405-409 (1995). Any appropriate vectors now known or later described for plant transformation are suitable for use with the present invention.

U.S. Patent No. 4,237,224 issued to Cohen and Boyer, describes the production of expression systems in the form of recombinant plasmids using restriction enzyme cleavage and ligation with DNA ligase. These recombinant plasmids are then introduced by means of transformation and replicated in unicellular cultures, including prokaryotic organisms and eukaryotic cells grown in tissue culture.

Once the DNA construct of the present invention has been cloned into an expression system, as described above, they are ready to be incorporated into a host cell. Recombinant molecules can be introduced into cells via transformation, particularly transduction, conjugation, mobilization, or electroporation. The DNA sequences are cloned into the vector using standard cloning procedures in the art, as described by Sambrook et al., Molecular Cloning: A Laboratory Manual, Second Edition, Cold Springs Laboratory, Cold Springs Harbor, New York (1989).

Suitable host cells include, but are not limited to, bacteria, virus, yeast, mammalian cells, insect, plant, and the like. Preferably, the host cells are either a bacterial cell (e.g., *Agrobacterium*) or a plant cell. Examples of plant cells include cells from cotton, corn, sugar cane, and rice stems that can be used in pulping, flax, hemp, ramie, jute, kenaf, kapok, coir, bamboq, spanish moss, abaca, and *Agave* spp. (e.g. sisal). Most preferably, the plant cell is from cotton.

In other embodiments of the present invention, plants or seeds are produced by transformation with the DNA construct of the present invention.

The present invention also relates to a method of imparting resistance to insects and fungi involving transforming a plant with the DNA construct that contains the chitinase-encoding nucleic acid molecule of the present invention. The DNA construct of the present invention can be utilized to impart resistance to insects and fungi for a wide variety of fiber-producing plants such as cotton, corn, sugar cane, and rice stems that can be used in pulping, flax, hemp, ramie, jute, kenaf, kapok, coir, bamboo, spanish moss, abaca, and *Agave* spp. (e.g. sisal). The DNA construct is particularly well suited to imparting resistance to cotton.

One approach to transforming plant cells with the nucleic acid molecule of the present invention is particle bombardment (also known as biolistic transformation) of the host cell. This can be accomplished in one of several ways. The first involves propelling inert or biologically active particles at cells. This technique is disclosed in U.S. Patent Nos. 4,945,050, 5,036,006, and 5,100,792, all to Sanford, et al.
Generally, this procedure involves propelling inert or biologically active particles at the cells under conditions effective to penetrate the outer surface of the cell and to be incorporated within the interior thereof. When inert particles are utilized, the vector can be introduced into the cell by coating the particles with the vector containing the heterologous DNA. Alternatively, the target cell can be surrounded by the vector so that the vector is carried into the cell by the wake of the particle. Biologically active particles (e.g., dried bacterial cells containing the vector and heterologous DNA) can also be propelled into plant cells. Other variations of particle bombardment, now known or hereafter developed, can also be used.

Transient expression in protoplasts allows quantitative studies of gene expression since the population of cells is very high (on the order of 10⁶). To deliver DNA inside protoplasts, several methodologies have been proposed, but the most common are electroporation (Fromm et al., "Expression of Genes Transferred Into Monocot and Dicot Plants by Electroporation," Proc. Natl. Acad. Sci, USA 82:5824-5828 (1985)) and polyethylene glycol (PEG) mediated DNA uptake (Krens et al., "In Vitro Transformation of Plant Protoplasts with Ti-Plasmid DNA," Nature 296:72-74 (1982)). During electroporation, the DNA is introduced into the cell by means of a reversible change in the permeability of the cell membrane due to exposure to an electric field. PEG transformation introduces the DNA by changing the elasticity of the membranes. Unlike electroporation, PEG transformation does not require any special equipment and transformation efficiencies can be equally high. Another appropriate method of introducing the gene construct of the present invention into a host cell is fusion of protoplasts with other entities, either minicells, cells, lysosomes, or other fusible lipid-surfaced bodies that contain the chimeric gene (Fraley, et al., "Liposome-Mediated Delivery of Tobacco Mosaic-Virus RNA Into Tobacco Protoplasts - A Sensitive Assay for Monitoring Liposome-Protoplast Interactions," Proc. Natl. Acad. Sci, USA, 79:1859-1863 (1982)),

Stable transformants are preferable for the methods of the present invention. An appropriate method of stably introducing the DNA construct into plant cells is to infect a plant cell with *Agrobacterium tumefaciens* or *Agrobacterium rhizogenes* previously transformed with the DNA construct. Under appropriate conditions known in the art, the transformed plant cells are grown to form shoots or roots, and develop further into plants. In one embodiment of the present invention, transformants are generated using the method of Frary et al, Plant Cell Reports 16: 235 (1996)) to transform seedling explants.

Plant tissues suitable for transformation include, but are not limited to, floral buds, leaf tissue, root tissue, hypocotyl tissue, meristems, zygotic and somatic embryos, megaspores, and anthers.

After transformation, the transformed plant cells can be selected and regenerated. Preferably, transformed cells are first identified using a selection marker simultaneously introduced into the host cells along with the DNA construct of the present invention. The most widely used reporter gene for gene fusion experiments has been *uid*A, a gene from *Escherichia coli* that encodes the β-glucuronidase protein, also known as GUS. Jefferson et al., "GUS Fusions: β Glucuronidase as a Sensitive and Versatile Gene Fusion Marker in Higher Plants," EMBO Journal 6:3901-3907 (1987)).
GUS is a 68.2 kd protein that acts as a tetramer in its native form. It does not require cofactors or special ionic conditions, although it can be inhibited by divalent cations like Cu²⁺ or Zn²⁺. GUS is active in the presence of thiol reducing agents like β-mercaptoethanol or dithiothreitol (DTT).

Other suitable selection markers include, without limitation, markers encoding for antibiotic resistance, such as the nptII gene which confers kanamycin resistance (Fraley, et al., "Expression of Bacterial Genes in Plant Cells,"Proc. Natl. Acad. Sci. USA, 80:4803-4807 (1983)) and the dhfr gene, which confers resistance to methotrexate (Bourouis et al., "Vectors Containing a Prokaryotic Dihydrofolate Reductase Gene Transform Drosophila Cells to Methotrexate-Resistance," EMBO J. 2:1099-1104 (1983)).

Once a recombinant plant cell or tissue has been obtained, it is possible to regenerate a full-grown plant therefrom. Means for regeneration vary from species to species of plants, but generally a suspension of transformed protoplasts or a petri plate containing transformed explants is first provided. Callus tissue is formed and shoots may be induced from callus and subsequently rooted. Alternatively, embryo formation can be induced in the callus tissue. These embryos germinate as natural embryos to form plants. The culture media will generally contain various amino acids and hormones, such as auxin and cytokinins. It is also advantageous to add glutamic acid and proline to the medium, especially for such species as corn and alfalfa. Efficient regeneration will depend on the medium, on the genotype, and on the history of the culture. If these three variables are controlled, then regeneration is usually reproducible and repeatable.

Plant regeneration from cultured protoplasts is described in Evans, et al., Handbook of Plant Cell Cultures, Vol, 1: (MacMillan Publishing Cb., New York, 1983); and Vasil I.R. (ed.), Cell Culture and Somatic Cell Genetics of Plants, Acad. Press, Orlando, Vol. I, 1984, and Vol. III (1986)).

It is known that practically all plants can be regenerated from cultured cells or tissues, including but not limited to, all major species of cotton, rice, wheat, barley, rye, sunflower, peanut, corn, potato, sweet potato, bean, pea, chicory, lettuce, endive, cabbage, cauliflower, broccoli, turnip, radish, spinach, onion, garlic, eggplant, pepper, celery, carrot, squash, pumpkin, zucchini, cucumber, apple, pear, melon, strawberry, grape, raspberry, pineapple, soybean, tobacco, tomato, sorghum, and sugarcane.

After the nucleic acid molecule of the present invention is stably incorporated in transgenic plants, it can be transferred to other plants by sexual crossing or by preparing cultivars. With respect to sexual crossing, any of a number of standard breeding techniques can be used depending upon the species to be crossed. Cultivars can be propagated in accord with common agricultural procedures known to those in the field. Alternatively, transgenic seeds are recovered from the transgenic plants. The seeds can then be planted in the soil and cultivated using conventional procedures to produce transgenic plants.

The present invention also relates to a method of regulating the cellulose content of a plant fiber involving transforming a plant with the DNA construct that contains the chitinase-encoding nucleic acid molecule of the present invention. Same approaches to transforming plant cells with the nucleic acid molecule of the present invention mentioned above could be used. The DNA construct of the present invention can be utilized to modulate fiber development by regulating cellulose synthesis for a wide variety of fiber-producing plants such as cotton, corn, sugar cane, and rice stems that can be used in pulping, flax, hemp, ramie, jute, kenaf, kapok, coir, bamboo, spanish moss, abaca, and *Agave* spp. (e.g. sisal). The DNA construct is particularly well suited to modulating fiber development of cotton.

Several kinds of evidence suggest that chitinases are located in healthy plant organs, and one developmental role of a chitinase in embryogenesis has been well-characterized. A carrot mutant defective in somatic embryogenesis can be rescued by addition of an acidic, glycosylated, extracellular, chitinase (EP3) to the culture medium (de Jong et al., "A Carrot Somatic Embryo Mutant is Rescued by Chitinase," Plant Cell, 4:425-433 (1992)).
The rescue,could be duplicated by addition of lipochitin oligosaccharides (Schmidt et al., "Signal Molecules Involved in Plant Embryogenesis," Plant Molecular Biology, 26:1305-1313 (1994)) and by some but not all other chitinases (Kragh et al., "Characterization of Chitinases Able to Rescue Somatic Embryos of the Temperature-Sensitive Carrot Variant TS11," Plant Molecular Biology, 31:631-645 (1996)).
The EP3 gene was expressed in a small subset of cells of young fruits and mature seeds, suggesting that the chitinase acts to release chitin oligomers as developmental signals (possibly to reintiate cell division) for developing zygotic embryos (van Hengel, "Expression Patterns of the Carrot EP3 Endochitinase Genes in Suspension Cultures and in Developing Seeds," Plant Physiology, 117:43-53 (1998)).
Chitinase gene expression has also been associated with somatic embryogenesis in other species (Dong et al., "Endochitinese and β-1,3-Glucanase Genes are Developmentally Regulated During Somatic Embryogenesis in Picea glauca," Planta, 201:189-194 (1997).
Arabinogalactan proteins (AGPs) are another class of molecules required for somatic embryogenesis, and some carrot AGPs contain glucosamine and N-acetyl-D-glucosaminyl and are sensitive to cleavage by chitinases. Pretreatment of AGPs with chitinase made them more active in promoting embryogenesis of carrot protoplasts, implying that embryo rescue by chitinase may be mediated by their hydrolytic action on AGPs (van Hengel et al., "N-Acetylglucosamine and Glucosamine-Containing Arabinogalactan Proteins Control Somatic Embryogenesis," Plant Physiology, 125:1880-1890 (2001)).
In this case, AGPs would be one endogenous substrate for chitinases. It has been found that an AGP-type protein accumulates during cotton fiber secondary wall deposition (John et al., "Characterization of mRNA for a Proline-Rich Protein of Cotton Fiber," Plant Physiology, 108:669-676 (1995)).

Chitinases have been associated with several other healthy plant tissues, including germinating seeds (Petruzzelli et al. "Distinct Ethylene- and Tissue-Specific Regulation of β-1,3-Glucanases and Chitinases During Pea Seed Germination," Planta, 209:193-201 (1999)).
Chitinase gene expression is associated with formation of flowers de novo from tobacco cell explants (Neale et al., " Chitinase, B-1,3-Glucanase, Osmotin, and Extensin are Expressed in Tobacco Explants During Flower Formation," Plant Cell, 2:673-684 (1990).
Chitinase enzyme activity has been found in healthy *Petunia* flowers, being particularly high in the stigma after the anthers dehisce (Leung, "Involvement of Plant Chitinases in Sexual Reproduction of Higher Plants," Phytochemistry, 31:1899-1900 (1992)).
Similarly, a protein immunologically related to basic chitinase was identified in both the anthers and the upper portion of bean pistils (containing the stigma) (del Campillo et al., "occurrence of 9.5 Cellulase and Other Hydrolases in Flower Reproductive Organs Undergoing Major Cell Wall Disruption," Plant Physiology, 99:1015-1020 (1992)).
In flowers, chitinase might have a developmental role or a defensive role in anticipation of possible fungal invasion of fragile tissues that are essential for reproductive success. Some proteins extracted from primary and secondary cell walls of various plant species in suspension culture and sequenced at their N-terminus have homology to chitinase sequences in the databases from French bean (P80800, P80808, P80792, P82432) and tobacco (P80783, P8233) (Robertson et al., "Differential Extraction and Protein Sequencing Reveals Major Differences in Patterns of Primary Cell Wall Proteins from Plants," The Journal of Biological Chemistry, 272:15841-15848 (1997); Blee et al., "Proteomic Analysis Reveals a Novel Set of Cell Wall Proteins in a Transformed Tobacco Cell Culture That Synthesizes Secondary Walls as Determined by Biochemical and Morphological Parameters," Planta, 212:404-415 (2001)).
However, since only short peptide sequences were available after N-terminal sequencing (maximum of eighteen amino acids), definite conclusions about the presence of authentic chitinases in these cell walls cannot be made.

Other than the N-acetyl-glucosamine-containing AGPs associated with embryo rescue (van Hengel et al., "N-Acetylglucosamine and Glucosamine-Containing Arabinogalactan Proteins Control Somatic Embryogenesis," f1mn Physiology, 125:1880-1890 (2001)). endogenous substrates of plant chitinases have not been characterized at the structural or functional levels. However, there is evidence that endogenous chitinase substrates of diverse molecular types exist in healthy plants. For example, a chitin-binding lectin, wheat germ agglutinin (WGA), or chitinase tagged with electron-dense colloidal gold labeled the vascular secondary cell walls very densely in healthy elm, tomato, eggplant, and potato plants. Adjacent primary walls or middle lamella regions were not labeled, which supports the specificity of the reaction (Benhamou et al., "Attempted Localization of Substrate for Chitinases in Plant Cells Reveals Abundant N-Acetyl-D-Glucosamine Residues in Secondary Walls," Biologie Cellulaire, 67:341- 350 (1989)).
This labeling could not be abolished by pre-treatment with protease to digest protein or a microbial chitinase, but it was abolished by pretreatment with lipase to digest lipids. The absence of label after lipase treatment would appear to place these secondary wall molecules in a different class than the N-acetyl-glucosanune-containing AGPs that have been associated with embryo rescue. Rather, the data suggest that chitin oligomers exist within or in association with a lipid-containing molecule in secondary cell walls. (0067) Molecules containing both chitin oligomers and lipid (a single fatty acyl group varying from C 16:0 to C20:4)) are known in the form of lipochitin oligosaccharides (LCOs), which are synthesized and secreted by nodulating bacteria (*Rhizobium*) to induce the formation of the symbiotic root nodule by their plant host (Bakkers et al., "Function of Chitin Oligosaccharides in Plant and Animal Development," in Jolles, eds., Chitin and Chitinases, Birkhäuser Verlag: Basel, pp.71-84 (1999)).
This indicates that plants have capacity to recognize such molecules supplied from an exogenous organism as developmental signals, in this case to initiate nodule formation. Furthermore, similar molecules may exist endogenously within plants. Thin layer chromatography (TLC) of lipophilic compounds in extracts from flowers of the plant *Lathyrus odoratus* showed that they migrated similarly to LCOs and that some of them were susceptible to chitinase digestion (Spaink et al., "Rhizobial Lipo-Oligosaccharide Signals and Their Role in Plant Morphogenesis: Are Analogous Lipophilic Chitin Derivatives Produced by the Plant?," Australian Journal of Plant Phyrsiology, 20:381-392 (1993)).

Endogenous plant molecules similar to LCOs could contain growth-regulating chitin oligomers that are releasable by endogenous plant chitinases. The data on embryo rescue by chitinase already described provide one example. Also, tomato suspension cells respond to LCOs or chitin oligomers (such as might be released from more complex endogenous molecules by limited chitinase action) by transiently raising the pH of their culture medium (Staehelin et al., "Perception of Rhizobium Nodulation Factors by Tomato Cells and Inactivation by Root Chitinases," Proc. Nat'l. Acad. Sci. U.S.A., 91:2196-2200 (1994)). Other plan responses associated with chitin oligomers include synthesis of anti-fungal phytoalexins, induction of chitinases, induction of K⁺ and Cl⁻ release, and H₂O₂ synthesis (Gooday, "Aggressive and Defensive Roles for Chitinases," in Jolles, eds., Chitin and Chitinases, Birkhäuser Verlag:Basel, pp. 157-170 (1999)). Tobacco plants transformed to over-express LCO-synthesizing genes (NodA and NodB, either separately or in combination) from *Rhizobium* had reduced growth and altered leaf shape (Schmidt et al., "Alteration of Plant Growth and Development by Rhizobium noda and Nodb Genes Involved in the Synthesis of Oligosaccharide Signal Molecules," The Plant Journal, 4:651-658 (1993)). suggesting that NodA and NodB can interfere with plant biosynthetic processes required for morphogenesis (Bakkers et al., "Function of Chitin Oligosaccharides in Plant and Animal Development," in Jolles, eds., Chitin and Chitinases, Birkhäuser Verlag: Basel, pp.71-84 (1999)).
Such interference could occur at many levels. However, since both overall growth rate and normal plant organ morphogenesis depend heavily on cellulose synthesis, it is possible that NodA and NodB interfere directly with cellulose synthesis. Perhaps NodA and NodB bind to and process inappropriately an endogenous N-acetyl-glucosamine-containing plant substrate, creating a non-functional analog of an endogenous molecule required for cellulose synthesis.

Conversely, tobacco plants transformed to over-express a maize acidic class I chitinase showed a positive correlation between chitinase activity and seedling dry weight (Patil et al., "Possible Correlation Between Increased Vigour and Chitinase Activity Expression in Tobacco," Journal of Experimental Botany, 48:1943-1950 (1997)), which has a large cellulose component. Although no mechanistic explanation was provided, it is possible that the foreign chitinase activity causes over-production of chitin oligomers that stimulate cellulose synthesis. Chitin oligomers could modulate cellulose synthesis either as developmental signals or as direct participants in the cellulose biosynthetic process. For example, oligomers of the N-acetylglucosamine-containing hyaluronan heteropolymer, (β-1,4-GlcA β-1,3-GlcNAc)ₙ, interact with a particular receptor to activate Rho and Rac1 GTPases, which lead to reorganization of the actin cytoskeleton (Lee et al., "Hyaluronan: A Multifunctional, Megadalton, Stealth Molecule," Current Opinion in Cell Biology, 12:581-586 (2000)).
Reorganization of the actin cytoskeleton occurs at the primary to secondary wall transition in cotton fibers to mediate the changed orientation of cellulose microfibrils (Seagull, "Cytoskeletal Involvement in Cotton Fiber Growth and Development," Micron, 24:643-660 (1993)).
A Rac gene is expressed transiently in cotton fibers at the time of this reorganization and (Delmer et al., " Genes Encoding Small GTP-Binding Proteins Analogous to Mammalian Rac are Preferentially Expressed in Developing Cotton Fibers," Mol. Gen. Genet., 248:43-51 (1995)) and a related H₂O₂ burst may also mediate this transition (Potikha et al., "The Involvement of Hydrogen Peroxide in the Differentiation of Secondary Walls in Cotton Fibers," Plant Physiology, 119: 849-858 (1999)).
However, the prolonged expression of the isolated nucleic acid molecule disclosed in the present invention throughout secondary wall deposition argues for a role beyond transient control of signal transduction cascades and cytoskeletal organization involving Rac and H₂O₂ at the primary to secondary wall transition.

Other possibilities for the involvement of the isolated nucleic acid molecule disclosed in the present invention in cotton fiber cellulose synthesis can be proposed by analogy with other systems. LCOs or similar molecules such as dolichol-(N-acetyl-glucosamine)ₙ could donate chitin-containing oligosaccharides to a protein as primers in a biosynthetic process (Spaink et al., "Rhizobial Lipo-Oligosaccharide Signals and Their Role in Plant Morphogenesis: Are Analogous Lipophilic Chitin Derivatives Produced by the Plant?," Australian Journal of Plant Physiology, 20:381-392 (1993)). as exemplified by chitin biosynthesis in insects (Palli et al., "Molecular and Biochemical Aspects of Chitin Synthesis Inhibition," in Jolles, eds., Chitin and Chitinases, Birkhäuser Verlag:Basel, pp. 85-98 (1999)).
It has been noted that little attention has been paid to the fact that two fungal chitinases and egg lysozyme act as catalysts in transglycosylation reactions (Collinge et al., "Plant Chitinases," Plant J., 3:31-40(1993)). *Arabidopsis* cytl mutants lack sufficient mannose-1-phosphate guanyltransferase activity, and consequently they are deficient in production of GDP-mannose as required for N-glycosylation of proteins. They also exhibit 5-fold reduction in cellulose content and cannot proceed through normal embryo development (Lukowitz et al., "Arabidopsis cyt1 Mutants are Deficient in a Mannose-1-Phosphate Guanyltransferase and Point to a Requirement of N-Linked Glycosylation for Cellulose Biosynthesis," Proc. Nat'l. Acad. Sci. U.S.A., 98:2262-2267 (2001), Since the N-glycosylation inhibitor, tunicamycin, also causes cellulose-deficiency, these authors suggest that an N-glycosylated peptide might act as a primer for cellulose synthesis, which is a resurrection of an old and much-debated hypothesis that cellulose synthesis does require a primer (Maclachlan, "Does β-Glucan Synthesis Need A Primer," in Brown, Jr., ed., Cellulose and Other Natural Polymer Systems: Biogenesis, Structure, and Degradation, Plenum Press:NY, pp. 327-339 (1982).

One form of protein-encoding DNA suitable for use in the DNA construct of the present invention is the isolated nucleic acid molecule of the invention, preferably, the nucleic acid molecule comprising a nucleotide sequence which hybridizes to a DNA molecule having a sequence according to SEQ. ID. No. 1 under stringent conditions characterized by a hybridization buffer comprising 1 x SSC at a temperature of 61°C, having a nucleotide sequence of SEQ. ID. No. 1, or encoding a protein or polypeptide having an amino acid sequence of SEQ. ID. No. 2.

Protein-encoding DNA suitable for use in the present invention includes DNA which has been amplified, chemically altered, or otherwise modified. Modification of such protein-encoding DNAs may occur, for example, by treating the DNA with a restriction enzyme to generate a DNA fragment which is capable of being operably linked to the promoter. Modification can also occur by techniques such as site-directed mutagenesis.

The protein-encoding DNA also includes DNA that is completely synthetic, semi-synthetic, or biologically derived, such as DNA derived by reverse transcription from RNA. Such DNA includes, but is not limited to, non-plant genes such as those from bacteria, yeasts, animals, or viruses; modified genes, portions of genes, chimeric genes, as well as DNA that encodes for amino acids that are chemical precursors or biologics of commercial value, such as polymers or biopolymers (Pool et al., "In Search of the Plastic Potato," Science, 245:1187-1189 (1989)). Suitable DNA is any DNA for which expression is beneficial to the transgenic plant or for which it is otherwise beneficial to have the DNA expressed under control of a DNA promoter that drives expression preferentially during the secondary wall stage of fiber development.

Examples of other protein-encoding DNA molecules that could be expressed in the present invention include, but are not limited to, homologous and heterologous cellulose synthases (*CesA* genes), both in normal and mutated form (Arioli et al., "Molecular Analysis of Cellulose Biosynthesis in Arabidopsis," Science, 279:717-720 (1998); Holland et al., "A Comparative Analysis of the Plant Cellulose Synthase (CesA) Gene Family," Plant Physiol., 123:1313-1324 (2000)), genes that may modulate carbon partitioning to cellulose (Delmer, "Cellulose Biosynthesis in Developing Cotton Fibers" in: A.S. Basra (ed.), Cotton Fibers: Developmental Biology, Quality Improvement, and Textile Processing, The Haworth Press, New York, pp. 85-112 (1999)); such as sucrose synthase (Amor et al., "A Membrane-Associated Form of Sucrose Synthase and Its Potential Role Synthesis of Cellulose and Callose in Plants," Proc. Natl. Acad. Sci. USA, 92:9353-9357 (1995)); sucrose phosphate synthase (Haigler et al., "Transgenic Cotton Over-Expressing Sucrose Phosphate Synthase Produces Higher Quality Fibers with Increased Cellulose Content and Has Enhanced Seed Cotton Yield" Abstract 477. In: Proceedings of Plant Biology 2000, July 15 - 19, San Diego, CA. American Society of Plant Physiologists, Rockville, MD, (2000), UDPG-pyrophosphorylase (Waffler and Meier, "Enzyme Activities in Developing Cotton Fibers," Plant Physiol. Biochem, 32:697-702 (1994)); inorganic pyrophosphosphatase (Geigenberger et al., "Overexpression of Pyrophosphatase Leads to Increased Sucrose Degradation and Starch Synthesis, Increased Activities of Enzymes for Sucrose-Starch Interconversions, and Increased Levels of Nucleotides in Growing Potato Tubers," Planta, 205:428-437(1998)), hexokinases (Smeekens, "Sugar Regulation of Gene Expression," Curr. Op. Plant Biol.,1:230-234(1998)), and invertases (Sturm and Tang, "The Sucrose-Cleaving Enzymes of Plants are Crucial for Development. Growth, and Carbon Partitioning," Trends Plant Sci., 4:401-407 (1999)); genes that might affect the molecular and biophysical properties of cellulose including degree of polymerization, degree of crystallinity, crystallite size, and microfibril orientation (i.e. genes for encoding proteins, including co-crystallizing protein polymers or cellulose binding domains, and polysaccharide-synthesizing and other enzymes) (Delmer, "Cellulose Biosynthesis: Exciting Times for a Difficult Field of Study," Ann. Rev. Plant Physiol. Mol. Biol. 50:245-276 (1999); Delmer, "Cellulose Biosynthesis in Developing Cotton Fibers. In: A.S. Basra (ed.), Cotton Fibers: Developmental Biology, Quality Improvement and Textile Processing, The Haworth Press, New York, pp. 85-112 (1999); Hsieh, "Structural Development of Cotton Fibers. In: A.S. Basra (ed.), Cotton Fibers: Developmental Biology, Quality Improvement, and Textile Processing, The Haworth Press, New York, pp. 137-166 (1999), transcription factors such as MYB genes that could prolong elongation growth and/or change the timing or extent of secondary wall deposition (Wilkins and Jernstedt, "Molecular Genetics of Developing Cotton Fibers. In: A.S. Basra (ed.), Cotton Fibers: Developmental Biology, Quality Improvement, and Textile Processing, The Haworth Press, New York, pp. 231-270 (1999), genes to effect the synthesis of plant hormones and change fiber properties (John, "Genetic Engineering Strategies for Cotton Fiber Modification. In: A.S. Basra (ed.), Cotton Fibers: Developmental Biology, Quality Improvement, and Textile Processing, The Haworth Press, New York, pp. 271-289 (1999)); genes for cytoskeletal elements or cytoskeletal-associated proteins that might affect fiber properties (Seagull, "Cytoskeletal Involvement in Cotton Fiber Growth and Development," Micron, 24:643-660 (1993)); genes for lipid synthesizing or modifying enzymes that might change membrane properties and thereby improve fiber quality, including under stressful environmental conditions (Haigler, "The Crystallinity of Cotton Cellulose in Relation to Cotton Improvement," Proc. Cotton Fiber Cellulose: Structure, Function, and Utilization Conference, National Cotton Council of America: Memphis, TN., p. 211-225 (1992)), enzymes such as xyloglucan endotransferase, peroxidase, expansin, or vacuolar ATPase that might, through increased or decreased activity, prolong or increase extension growth during secondary wall deposition (Wilkins and Jernstedt, "Molecular Genetics of Developing Cotton Fibers. In: A.S. Basra (ed.), Cotton Fibers: Developmental Biology. Quality Improvement, and Textile Processing, The Haworth Press, New York, pp. 231-270 (1999)); genes for protein or plastic polymers that might be retained in the fiber lumen or integrated into the cell wall to increase fiber strength or change its textile properties (John, "Genetic Engineering Strategies for Cotton Fiber Modification," In: A.S. Basra (ed.). Cotton Fibers: Developmental Biology, Quality Improvement, and Textile Processing, The Haworth Press, New York, pp. 271 - 289 (1999); Guda et al., "Hyperexpression of an Environmentally Friendly Synthetic Polymer Gene," Biotechnology Letters. 17:745-750 (1995)); genes for plant cell wall matrix biosynthetic enzymes or their regulatory proteins so that other carbohydrates could be integrated into the cell wall and change fiber properties (Haigler, "The Relationship Between Polymerization and Crystallization in Cellulose Biogenesis," in C. H. Haigler and P. Weimer, eds., Biosynthesis and Biodegradation of Cellulose, New York: Marcel Dekker, pp. 99-124 (1991); Andrawis et al., "Cotton Fiber Annexins: A Potential Role in the Regulation of Callose Synthase," Plant J., 3: 763-772 (1993)); genes for molecules such as tannins, suberins, or dyes that might confer valuable color to fibers (Ryser, "Cotton Fiber Initiation and Histodifferentiation," In: A.S. Basra (ed.), Cotton Fibers: Developmental Biology, Quality Improvement, and Textile Processing, The Haworth Press, New York, pp. 1-46 (1999), which is hereby incorporated by reference in its entirety); genes for molecules such as cutin, suberin, or wax that might change the absorptivity and strength of cotton fibers (May, "Genetic Variation in Fiber Quality," In: A.S. Basra (ed.), Cotton Fibers: Developmental Biology, Quality Improvement, and Textile Processing, The Haworth Press, New York, pp. 183-230 (1999); Ryser, "Cotton Fiber Initiation and Histodifferentiation," In: A.S. Basra (ed.), Cotton Fibers: Developmental Biology. Quality Improvement, and Textile Processing, The Haworth Press, New York, pp. 1-46 (1999)); and genes for signal transduction molecules such as Rac that may regulate shifts between fiber developmental stages (Delmer et al., "Genes Encoding Small GTP-Binding Proteins Analogous to Mammalian rac are Preferentially Expressed in Developing Cotton Fibers," Mol. Gen. Genet., 248: 43-51 (1995)),

The DNA construct of the present invention also includes an operable 3' regulatory region, selected from among those which are capable of providing correct transcription termination and polyadenylation of mRNA for expression in plant cells, operably linked to the a DNA molecule which encodes for a protein of choice. A number of 3' regulatory regions are known to be operable in plants. Exemplary 3' regulatory regions include, without limitation, the nopaline synthase 3' regulatory region (Fraley, et al., "Expression of Bacterial Genes in Plant Cells," Proc. Natl Acad. Sci. USA, 80:4803-4807 (1983)) and the cauliflower mosaic virus 3' regulatory region (Odell, et al., "Identification of DNA Sequences Required for Activity of the Cauliflower Mosaic Virus 35S Promoter," Nature, 313(6005):810-812(1985)). Virtually any 3' regulatory region known to be operable in plants would suffice for proper expression of the coding sequence of the DNA construct of the present invention.

The protein-encoding DNA molecule, the promoter of the present invention, and a 3' regulatory region can be ligated together using well known molecular cloning techniques as described in Sambrook et al., Molecular Cloning: A Laboratory Manual, Second Edition, Cold Spring Harbor Press, NY (1989).

In another embodiment, the present invention is an expression system that includes a suitable vector containing the above DNA construct of the invention. The present invention also includes host cells which include the DNA construct of the invention described above, transgenic plants and seeds produced

### EXAMPLES

### Example 1 - Identification of F285, a cDNA Clone Preferentially Expressed in Fibers During Secondary Wall Deposition

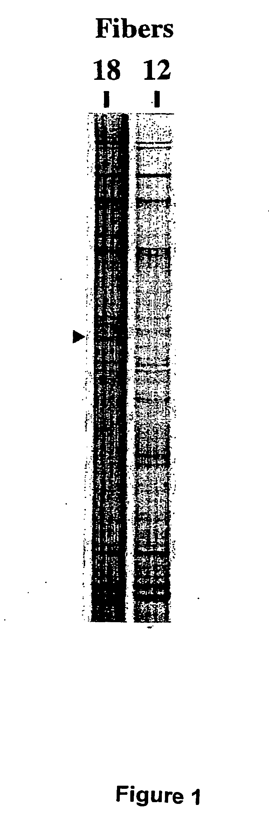

By differential display comparing 12 and 18 DPA cotton fibers (as well as stripped ovules and fibers at several other ages), a cDNA fragment (F285) that appeared to be preferentially expressed in fibers during secondary wall deposition was identified (Figure 1). In the differential display procedure, d(T)₁₂-GA was used as the anchored primer and GTCCTGGGTT as the random 10-mer. After cloning and partial sequencing, the cDNA fragment was found to be homologous to known chitinases as described later.

### Example 2 - Expression of F285 in Fibers During Secondary Wall Deposition

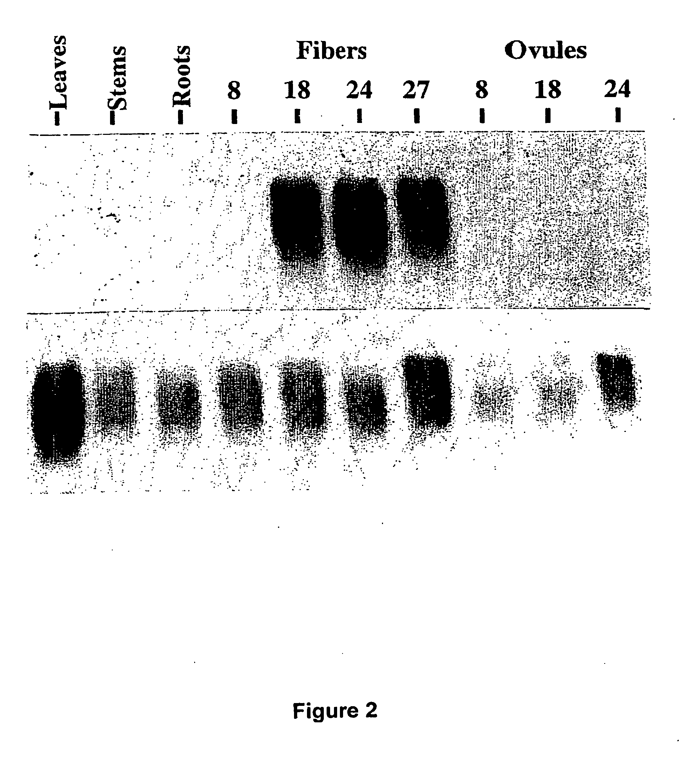

Northern blot analysis (overnight exposure to film) of cotton tissues indicated that the gene F285 is expressed in greenhouse-grown fibers only at the secondary wall stage at 18, 24, and 27 DPA (Figure 2). There was no evidence of expression in fibers during primary wall deposition at 8 DPA, or in leaves, stems, roots, or ovules stripped of fiber at 8, 18, and 24 DPA. (The data from stripped ovules at 8 and 18 DPA was not as good because of the lower level of RNA loading. However, any strong expression similar to that shown for 18, 24, and 27 DPA fiber would have been apparent even with the lower loading.)

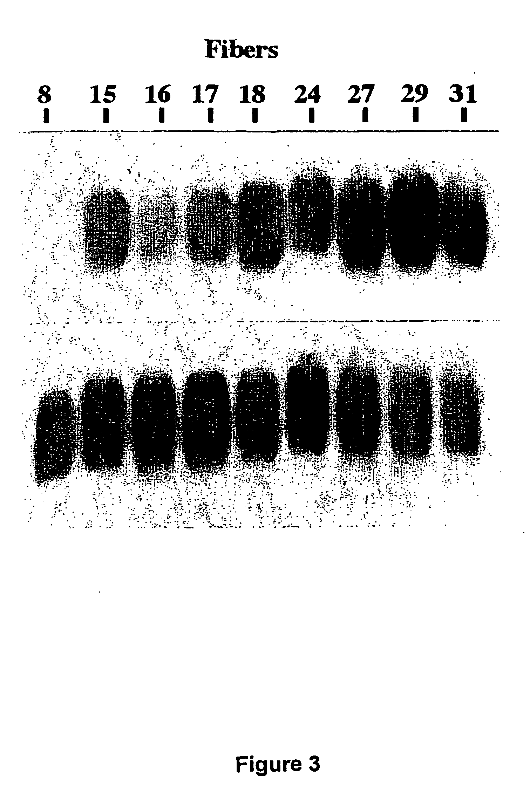

More detailed temporal analysis indicated that expression in greenhouse-grown fiber had not begun by 12 DPA, but was strong by 15 DPA and remained strong through 31 DPA (Figure 3). Therefore, the transcription of F285 is initiated at 13-15 DPA, which is near the onset of secondary wall deposition in cotton fiber when cotton plants are grown under greenhouse conditions (approximately 32°C day/22°C night).

### Example 3 - Sequencing of F285 and Comparison of its Translated Amino Acid Sequence to Other Proteins

PCR screening of a cDNA library from 21 DPA Acala-SJ2 cotton fibers (kindly provided by Dr. Deborah P. Delmer and The Hebrew University of Jerusalem) identified 3 similar length clones. Complete and repeated sequencing of these resolved them to one full-length sequence with 957 nucleotides and one open reading frame. The deduced amino acid sequence of 318 residues has a predicted pI of 7.98 and a predicted molecular weight of 35,246 Da. The translated protein has a diverse mix of amino acids, a predicted N-terminal signal sequence, and sites for post-translational modification by: N-glycosylation, phosphorylation by four different types of protein kinases; amidation; and N-myristoylation. The F285 protein has a mix of hydrophilic and hydrophobic regions, extensive regions of alpha helix, extended strand, and random coil, and no predicted transmembrane alpha helices.

BLASTX searches that translated the F285 nucleotide sequence in all possible reading frames and checked for homology with other amino acid sequences showed homology with numerous chitinases. The most recent BLASTX search (4/9/01) confirmed that there are only two close homologs of F285 that were discovered through sequencing of the *Arabidopsis thaliana* genome, one on chromosome 3 (BAA94976.1) and one on chromosome 1 (AAF29391.1). Hereafter, these are referred to as the *Arabidopsis* homologs of F285. The next closest match was to a chitinase from *Arabis microphylla* (AAF69789.1) (Bishop et al., "Rapid Evolution in Plant Chitinases: Molecular Targets of Selection in Plant-Pathogen Coevolution, Proc, Nat'l. Acad. Sci. U.S.A., 97:5322-5327 (2000)).

The BLAST search also confirmed that F285 was similar to but distinct from full-length or nearly full-length cotton chitinases (Q39799, Q39785, AAD11255, and S72528, where the first three sequences listed are very closely related) in the databases (see TABLE I). A BLASTP search with the F285 translated amino acid sequence did not reveal any additional highly similar sequences, except that another *Arabis* sequence closely related to AAF69789.1 (Bishop et al., "Rapid Evolution in Plant Chitinases: Molecular Targets of Selection in Plant-Pathogen Coevolution, Proc. Nat'l. Acad. Sci. U.S.A., 97:5322-5327 (2000)) was placed as the third highest match.

The amino acid sequences of BAA94976.1, AAF69789.1, and Q39799 were subjected individually to CLUSTAL W multiple sequence alignment with the F285 amino acid sequence to compare the identity, similarity, or difference of amino acids at each position (TABLE 1). F285 is much more similar to its *Arabidopsis* homolog (BLAST score 542; 76.28% identical amino acids) than to the chitinase from *Arabis* (BLAST score 212; 35.22% identical amino acids) or cotton (BLAST score 196; 31.66% identical amino acids). Since the genus *Arabis* contains the nearest relatives of *Arabidopsis thaliana* (Bishop et al., "Rapid Evolution in Plant Chitinases: Molecular Targets of Selection in Plant-Pathogen Coevolution, Proc. Nat'l. Acad. Sci. U.S.A., 97:5322-5327 (2000)) the comparative distance between these four sequences is the first indication that F285 and its *Arabidopsis* homolog represent a new type of chitinase-related protein.

**TABLE 1**

| **Results from CLUSTAL W multiple sequence alignment and BLASTX data** | | | | | | |
|---|---|---|---|---|---|---|
| | CLUSTAL W amino acid comparisons | | | | BLASTX data | |
| F285 (318 aa) compared to: | Identical | Strongly Similar | Weakly Similar | Different | Score | E |
| Best Match, | | | | | | |
| *Arabidopsis*/BAA94976.1 | | | | | | |
| (333 aa) | 76.28% | 9.91% | 3.60% | 10.21% | 542 | e-153 |
| Closest other genus, | | | | | | |
| *Arabis microphyllal*/ | | | | | | |
| AAF697B9.1 | | | | | | |
| (299 aa; partial sequence) | 35.22% | 19.18% | 11.01% | 34.59% | 212 | 6e-54 |
| Closest cotton, | | | | | | |
| *Gossypium hirsutum*/Q39799 | | | | | | |
| (338 aa) | 31.66% | 20.12% | 11.54% | 36.69% | 196 | 4e-49 |

F285 has homology with chitinases in regions corresponding to the active site (within 0.6 nm of bound substrate; Brameld et al., "The Role of Enzyme Distortion in the Single Displacement Mechanism of Family 19 Chitinases," Proc. Nat'l. Acad. Sci. U.S.A., 95:4276-4281 (1998)), including at a site (near NYNY) that is important for chitin binding in many chitinases (Verburg et al., "Identification of an Essential Tyrosine Residue in the Catalytic Site of a Chitinase Isolated From Zea mays That is Selectively Modified During Inactivation With 1-ethyl-3-(3-dimethylaminopropyl)-carbodiimide," J. Biol, Chem., 267:3886-3893 (1992); Hamel et al., "Structural and Evolutionary Relationships Among Chitinases of Flowering Plants," J. Mol. Evol., 44:614-624 (1997); Bishop et al., "Rapid Evolution in Plant Chitinases: Molecular Targets of Selection in Plant-Pathogen Coevolution, Proc. Nat'l. Acad. Sci. U.S.A., 97:5322-5327 (2000) TABLE 2). Chitin is a. structural polysaccharide, a homopolymer of β-1,4-linked N-acetyl glucosamine, that is found in the exoskeletons of insects and the cell wall of fungi (Hamel et al., "Structural and Evolutionary Relationships Among Chitinases of Flowering Plants," J. Mol. Evol., 44:614-624 (1997)).
However, here 'chitin binding' refers to binding of the enzyme with the short stretches (probably 3 - 6 sugars) of N-acetyl-glucosamine residues that interact with the enzyme during chitin hydrolysis (Robertus et al., "The Structure and Action of Chitinases," in Jolles, eds., Chitin and Chitinases, Birkhäuser Verlag:Basel, pp. 125-136 (1999))_{.}
Therefore, an enzyme with similar active site topology could bind with N-acetyl-glucosamine oligomers found in other molecules or in isolation. Also, it could interact with other molecules containing N-acetyl-glucosamine within heteropolymers or glycoproteins. As an example, some chitinases have lysozyme activity (Sahai et al., "Chitinases of Fungi and Plants: Their Involvement in Morphogenesis and Host-Parasite Interaction," FEMS Microbiol, Rev., 11:317-338 (1993,), meaning the ability to degrade bacterial peptidoglycan, which contains a heteropolymer of N-acetylglucosamine and N-acetylmuramic acid.

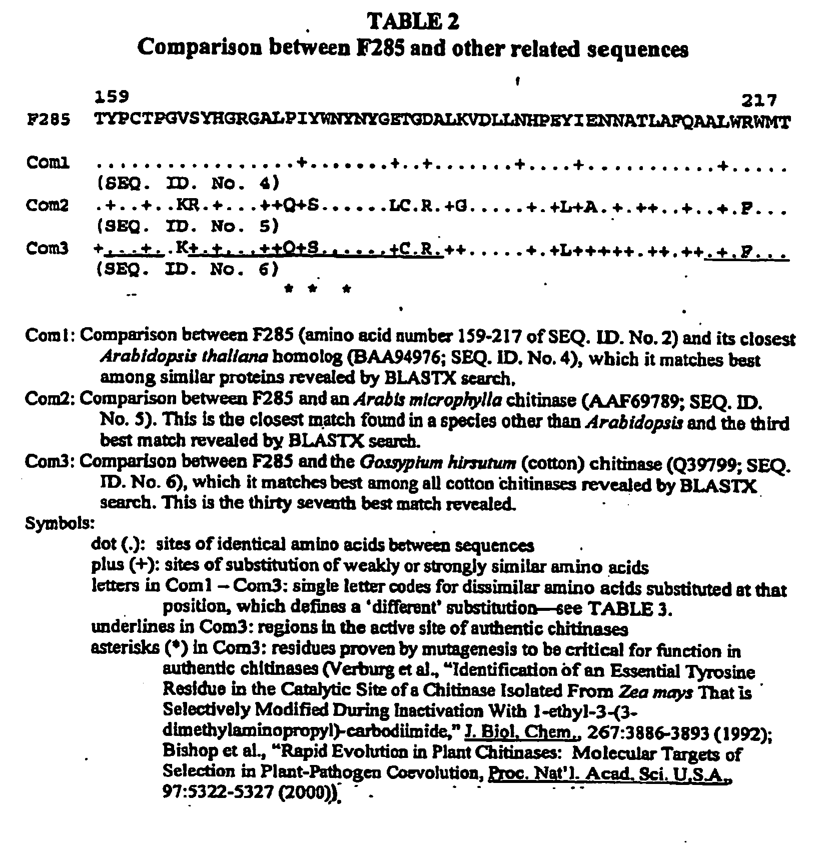

The *Arabis* protein listed in TABLES 1 and 2 is homologous to *Arabidopsis* class I chitinase, which has proven chitinolytic activity and defensive activity against fungi (Bishop et al., "Rapid Evolution in Plant Chitinases: Molecular Targets of Selection in Plant-Pathogen Coevolution, Proc. Nat'l, Acad. Sci. U.S.A., 97:5322-5327(2000)).
However, no function has been proven for F285 or its *Arabidopsis* homologs. After appearing in the database as part of the completely-sequenced *Arabidopsis* genome, the *Arabidopsis* homologs of F285 were categorized with chitinases in Family 19 of glycosyl hydrolases (CAZy, Carbohydrate-Active Enzymes; http://afmb.cnrs-mrs.fr/∼pedro/CAZY/db.html). Enzymes in Family 19 configuration (Henrissat, "Classification of Chitinase Modules," in Jolles, eds., Chitin and Chitinases, Basel:Birkhaüser Verlag, pp. 137-156 (1999)).
However, in the context of overall similarity to chitinases, specific differences in functional domains can be identified between F285 and its two *Arabidopsis* homologs vs. authentic chitinases. For example, F285 and its *Arabidopsis* homologs lack features that are typical of some, but not all, chitinases: (a) a cysteine-rich N-terminal chitin binding domain that would facilitate interaction with chitin; (b) a P (proline)/T (threonine)-rich hinge region before the catalytic region; and (c) a C-terminal vacuolar targeting domain (Meins et al., "The Primary Structure of Plant Pathogenesis-Related Glucanohydrolases and Their Genes," in Boller, eds., Genes Involved in Plant Defense, Springer Verlag/New York, p. 245-282 (1992)).
In the absence of the vacuolar targetting domain, F285 is expected to be secreted outside the plasma membrane. Its myristoylation site also confers potential for acylation by the covalent addition of myristate (a C14-saturated fatty acid), which could facilitate reversible interaction with the plasma membrane (Thompson et al., "Lipid-Linked Proteins of Plants," Prog. Lipid Res., 39:19 - 39 (2000)).

In addition, F285 and its *Arabidopsis* homologs have non-conservative amino acid substitutions (i.e. with a different or dissimilar chemical type of amino acid) in regions proved by mutagenesis to be critical for the function of typical chitinases (Iseli-Gamboni et al., "Mutation of Either of Two Essential Glutamates Converts the Catalytic Domain of Tobacco Class I Chitinase Into a Chitin-Binding Lectin," Plant Sci., 134:45-51 (1998), and in other regions predicted by crystal structure to be important for catalysis, active site geometry, or substrate binding (Hart et al., "The Refined Crystal Structure of an Endochitinase From Hordeum vulgare L. Seeds at 1.8 A Resolution," J, Mol, Biol., 248:402-413 (1995); Hamel et al., "Structural and Evolutionary Relationships Among Chitinases of Flowering Plants," J. Mol. Evol., 44:614-624 (1997); Bishop et al., "Rapid Evolution in Plant Chitinases: Molecular Targets of Selection in Plant-Pathogen Coevolution, Proc. Nat'l. Acad. Sci. U.S.A., 97:5322-5327 (2000)). For example, of eleven amino acids in an *Arabis* chitinase that are putative substrate binding sites (Bishop et al., "Rapid Evolution in Plant Chitinases: Molecular Targets of Selection in Plant-Pathogen Coevolution, Proc. Nat'l. Acad. Sci. U.S.A., 97:5322-5327 (2000)), two are unchanged, three are substituted with similar amino acids, and six are substituted with dissimilar amino acids in F285. This suggests that F285, while related to chitinases, might have unique protein structure allowing a unique cellular role. Some of these non-conservative changes and, conversely, amino acids that were retained as expected for chitinases in the sequences of F285 and its *Arabidopsis* homologs, are summarized in TABLE 3 (compiled from Hamel et al., "Structural and Evolutionary Relationships Among Chitinases of Flowering Plants," J. Mol. Evol., 44:614-624 (1997); Bishop et al., "Rapid Evolution in Plant Chitinases: Molecular Targets of Selection in Plant-Pathogen Coevolution, Proc. Nat'l. Acad. Sci. U.S.A., 97:5322-5327 (2000)).
Amino acids with asterisks (*) have been proven by mutagenesis to be important for the function of authentic chitinases (Iseli-Gamboni et al., "Mutation of Either of Two Essential Glutamates Converts the Catalytic Domain of Tobacco Class I Chitinase Into a Chitin-Binding Lectin," Plant Sci., 134:45-51 (1998)).
The sequence of an *Arabis* chitinase has been used for the reference amino acid number system because of its prior annotation in the literature (Bishop et al., "Rapid Evolution in Plant Chitinases: Molecular Targets of Selection in Plant-Pathogen Coevolution, Proc. Nat'l. Acad. Sci. U.S.A., 97:5322-5327 (2000)).
When amino acid substitutions are shown, the same ones were found at that position in F285 and its *Arabidopsis* homologs, except for an exception for AAF29391.1 as noted in the footnotes. 'Type of substitution' is as defined in the CLUSTAL W multiple sequence alignment program.

**TABLE 3**

| **Comparison of functional amino acids in chitinases to the F285 protein sequence** | | | | |
|---|---|---|---|---|
| *Arabis* Location | F285 Location | Expected Amino acid | Amino acid Found | Type of Substitution |
| 141 | 122 | *Glutamic acid (E) | Lysine (K) | Strongly similar Strongly similar |
| 142 | 123 | Threonine (T) | Same | |
| 163 | 144 | *Glutamic Acid (E) | Same | |
| 177 | 160 | Tryptophan (W)¹ | Tyrosine (Y) | Strongly similar |
| 192 | 175 | *Glutamine (Q) | Proline (P) | Different |
| 194 | 177 | *Serine (S) | Tyrosine (Y) | Different |
| 197 | 180 | *Tyrosine (Y)² | Same (in NYNY) | |
| 198 | 181 | Asparagine (N) | Same (in NYNY) | |
| 273 | 257 | *Asparagine (N) | Tyrosine (Y) | Different |

| | | | | |
|---|---|---|---|---|
| *Amino acids proven by mutagenesis to be required for chitinase activity (Iseli-Gamboni et al., "Mutation of Either of Two Essential Glutamates Converts the Catalytic Domain of Tobacco Class I Chitinase Into a Chitin-Binding Lectin," Plant Sci., 134:43-51 (1998)). ¹Retained as W in AAF29391.1 and found as Y in 3 of 4 cotton chitinases (Q39799, Q39785, and AAD11255). Other comparisons of F285 with all 4 cotton chitinases in the databases are consistent with the table. ²Changed to F in AAF29391.1, the other *Arabldopsis* homolog of F285. | | | | |

The difference between F285, its closest *Arabidopsis* homolog, and other chitinases is emphasized by comparing their similar sequences near NYNY (a substrate binding site) to a putative consensus sequence described for several classes of chitinases (Hamel et al., "Structural and Evolutionary Relationships Among Chitinases of Flowering Plants," J. Mol. Evol., 44:614-624 (1997)) and with the *Arabis* chitinase (AAF69789.1) as a representative of the consensus-type sequence (Bishop et al., "Rapid Evolution in Plant Chitinases: Molecular Targets of Selection in Plant-Pathogen Coevolution, Proc. Nat'l. Acad. Sci. U.S.A., 97:5322-5327 (2000))
In this case, gaps inserted in all the sequences, including the putative consensus sequence, may create the best mutual alignment. As seen in TABLE 4, the sequence of F285 and its *Arabidopsis* homolog could be viewed in comparison to the consensus and the *Arabis* sequence as: (a) adding Y with the result that the hydrophobic domain containing NYNY is lengthened; (b) deleting QLS with the result that a conserved P (in 38 of 39 listed chitinase sequences, Hamel et al., "Structural and Evolutionary Relationships Among Chitinases of Flowering Plants," J. Mol. Evol., 44:614-624 (1997)) is nearer the NYNY domain; and (c) adding AL with the result that the conserved GRG (in 38 of 39 listed chitinase sequences, Hamel et al., "Structural and Evolutionary Relationships Among Chitinases of Flowering Plants," J. Mol. Evol., 44:614-624 (1997)) remains 5 residues from the NYNY domain. Although this reasoning is hypothetical, it emphasizes the difference between the sequences of F285 and its *Arabidopsis* homolog compared to previously described chitinase sequences. Both the Q and the S are proven to be essential for catalysis in some chitinases (Hamel et al., "Structural and Evolutionary Relationships Among Chitinases of Flowering Plants," J. Mol. Evol., 44:614-624 (1997); Bishop et al., "Rapid Evolution in Plant Chitinases: Molecular Targets of Selection in Plant-Pathogen Coevolution, Proc. Nat'l. Acad. Sci. U.S.A., 97:5322-5327 (2000)), and either their substitution with different amino acids (TABLE 2 and 3) or hypothetical deletion (TABLE 4) may cause the function of F285 and its *Arabidopsis* homolog to diverge from that typically expected of chitinases. Alternatively, F285 and its *Arabidopsis* homologs could be a previously unrecognized type of chitinase with a unique cellular role.

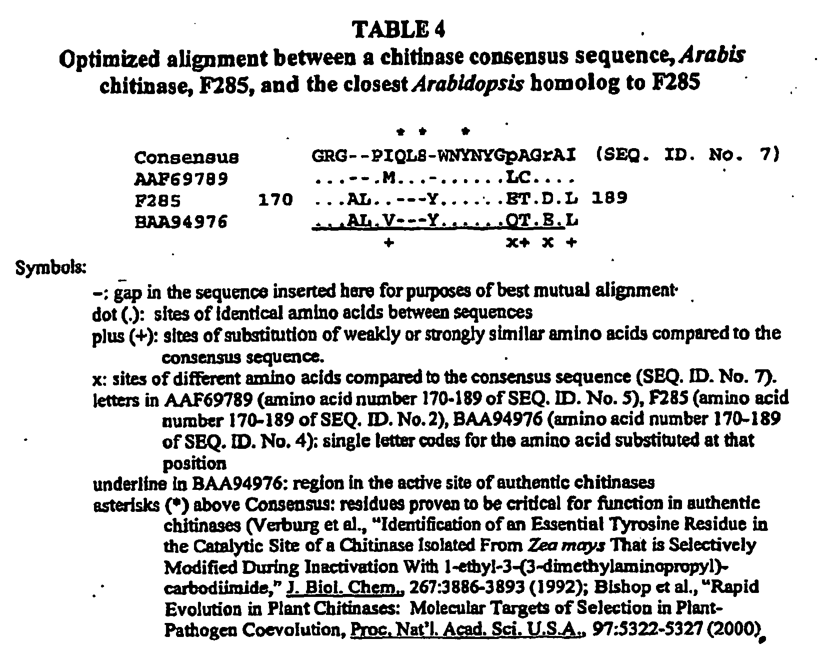

A similar divergence between F285 and its *Arabidopsis* homolog with other Family 19 chitinases is observed in a region previously defining part of a 'Signature' containing regions of putatively unchanging amino acids (underlined) in this family of glycosyl hydrolases (Robertus et al., "The Structure and Action of Chitinases," in Jolles, eds., Chitin and Chitinases, pp. 125-136 (1999); TABLE 5). The Signature contains two glutamic acid residues (E; at positions 122 and 144 of F285) that have been proven by mutagenesis to be catalytic residues (Iseli-Gamboni et al., "Mutation of Either of Two Essential Glutamates Converts the Catalytic Domain of Tobacco Class I Chitinase Into a Chitin-Binding Lectin," Plant Sci., 134:45-51 (1998)).
In F285 and its *Arabidopsis* homolog, the putatively conserved catalytic E at position 122 is substituted with K, which is a very similar amino acid although it is positively charged at pH 6 whereas E is negatively charged and it has an amino terminus of its R group whereas E has a carboxy terminus. Substitution by targeted mutagenesis of this E with A (a different type of amino acid) abolishes catalytic activity in some chitinases, changing the protein into a chitin-binding lectin (Iseli-Gamboni et al., "Mutation of Either of Two Essential Glutamates Converts the Catalytic Domain of Tobacco Class I Chitinase Into a Chitin-Binding Lectin," Plant Sci., 134:45-51 (1998).
However, the effect of change to K in F285 is unknown, although substitution with similar amino acids can cause changes in binding interactions of chitinases (Bishop et al., "Rapid Evolution in Plant Chitinases: Molecular Targets of Selection in Plant-Pathogen Coevolution, Proc. Nat'l. Acad. Sci. U.S.A., 97:5322-5327 (2000).
There are also many other examples of substitution of similar amino acids within the putatively unchanging signature residues. In addition, within the putatively unchanging signature residues, there are three sites (denoted by x) of substitution of different amino acids in F285 and its *Arabidopsis* homolog. These are more likely to have major functional consequences than the substitutions of similar amino acids. Note that the sequence of F285 and its *Arabidopsis* homolog are identical in this region, suggesting that the changes they both show compared to the Signature are important for their particular function. In contrast, the sequence of the *Arabis* chitinase (AAF69789) with a defensive role (Bishop et al., "Rapid Evolution in Plant Chitinases: Molecular Targets of Selection in Plant-Pathogen Coevolution, Proc. Nat'l. Acad. Sci. U.S.A., 97:5322-5327 (2000) matches the regions of unchanging amino acids in the Signature exactly and has only similar substitutions at other locations.

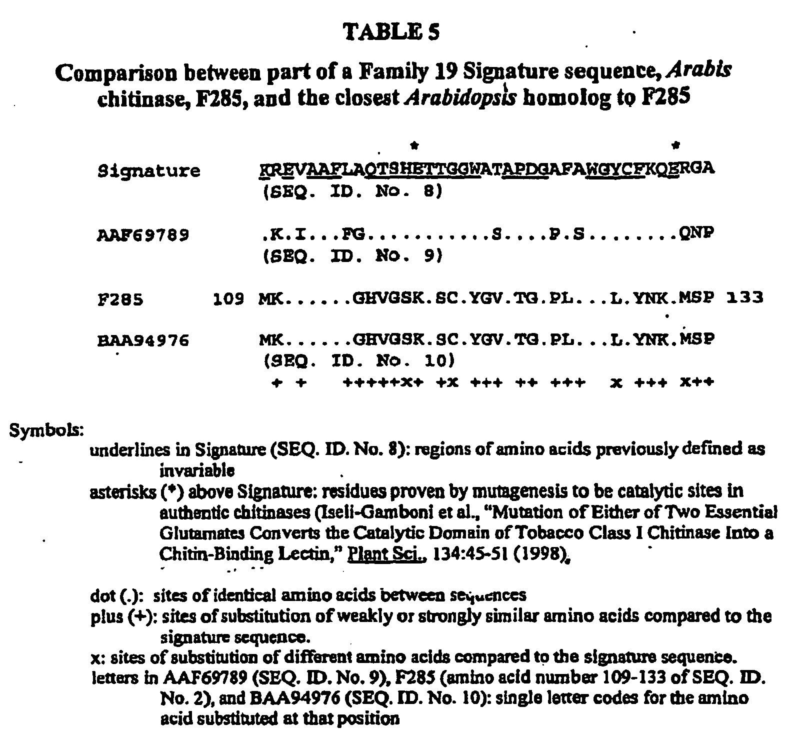

In summary, the data show that F285 and its closest *Arabidopsis* homolog are much more similar to each other than to the many chitinases in the sequence databases. The data allow the possibility that F285 and its *Arabidopsis* homologs have a function somewhat different than previously described for chitinases. The change in protein structure near the substrate binding sites and in the catalytic region are likely to be important for the special function and/or optimal function of F285 and its *Arabidopsis* homologs. For example, these proteins might interact with N-acetyl-glucosamine oligomers or heteropolymers found in as yet unidentified substrate molecules. Possible substrate molecules could include signaling molecules or glycoproteins containing homo- or hetero-oligomers of N-acetylglucosamine, not only structural chitin in insects or fungi (Sahai et al., "Chitinases of Fungi and Plants: Their Involvement in Morphogenesis and Host-Parasite Interaction," FEMS Microbiology Rey., 11:317-338(1993) .

The fact that F285 was discovered by analyzing changes in gene expression in the context of a particular developmental event (the initiation and continuation of secondary wall deposition in cotton fibers) and the *Arabidopsis* homologs of F285 were discovered only by complete sequencing of the *Arabidopsis* genome suggest that these chitinase-related proteins are more rarely expressed in the plant. This possibility is consistent with data from Northem blots of various cotton tissues (Figure 2). The high, cross-species, amino acid identity of F285 and its closest *Arabidopsis* homolog (76% identical; TABLE 1) intuitively suggest that these proteins have a critical role, possibly a developmental role, in the life of the plant. In contrast, chitinases such as are encoded by *Arabis* AAF69789.1 and *Gossypium* Q39799, which are more dissimilar to F285, have been most clearly associated with defensive roles. The existence of the *Arabidopsis* homologs of F285 opens efficient avenues to test the protein function via screening stocks of *Arabidopsis* insertional mutants be standard molecular biological techniques. In addition, gene expression could be down-regulated in *Arabidopsis* or cotton by anti-sense or RNAi technology that are standard molecular biology techniques.

### Example 4 - Assay of Chitinase Activity

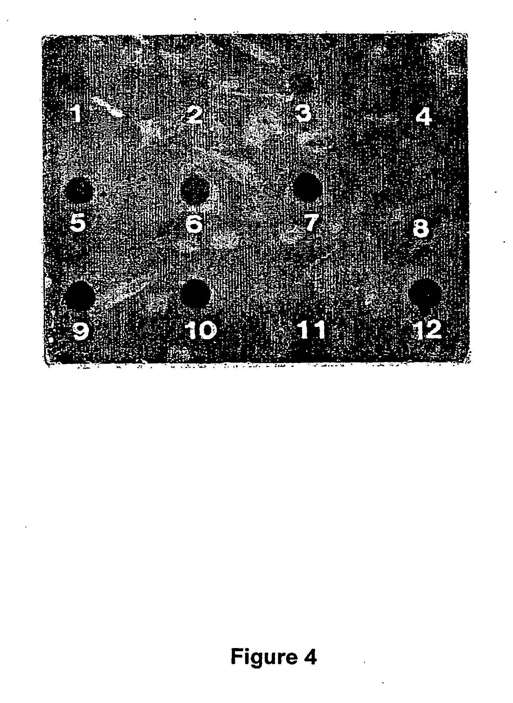

To begin to explore the biological role of the cognate protein of F285, chitinase activity was assayed in greenhouse-grown and cultured cotton fibers and seedling leaf blades in the presence and absence of salicylic acid (SA) and ethephon (ETH), an ethylene- producing compound. SA and ETH were used to spray leaves or cultured cotton fibers, because these compounds induce chitinases in many other systems (Cutt et al., "Pathogenesis-Related Proteins," in Boller eds., Genes Involved in Plant Defense, New York, New York:Springer-Verlag/Wien, pp. 209-243 (1992).
Seedlings were sprayed with SA, ETH, or water (as a control) 16 days after planting and leaves and stems were harvested 2 days later. Ovules cultured on 1 DPA were removed from culture flasks on 6 DPA, sprayed similarly, and replaced in flasks, followed by fiber harvesting on 8 DPA. Droplets of tissue extracts (10 µg total protein; extracted per methods of Mauch et al., "Antifungal Hydrolases in Pea Tissue. I. Purification and Characterization of Two Chitinases and Two β-1,3-Glucanases Differentially Regulated During Development and in Response to Fungal Infection." Plant Physiology, 87: 325-333 (1998) or control solutions were applied to areas (indicated by 1-12 in Figure 4) on a polyacrylamide gel containing suspended glycol chitin, and incubation to allow enzyme activity was carried out for 1 h at 37°C. A fluorescent brightener, Tinopal LPW, with high affinity for polymeric chitin was applied, and the gel was excited with UV light Subsequently, chitinase activity in tissue extracts was detected by measuring the ability to clear (degrade) water-soluble glycol chitin suspended in the polyacrylamide gel (Trudel et al., "Detection of Chitinase Activity After Polyacrylamide Gel Electrophoresis," Anal. Biochem., 178:362-366 (1989).
Dark areas indicate that the polymeric chitin in that location was degraded so that the fluorescent dye no longer bound to it. Therefore, dark areas demonstrate chitinase activity in the applied solution.

The positive control (area 12) showed strong chitinase activity. The negative controls (areas 8 and 11) showed no chitinase activity. All the cotton tissues tested showed chitinase activity, and no SA-induction of enzyme activity was observed by this qualitative test

### Example 5 - Induction of F285 Expression in Fibers by SA (for reference only)

Northern blots were performed to indicate whether or not the F285 promoter was responsive to the same inductive signals of many other plant chitinases (Figure 5). Treatment with water was used as a negative control. Fiber was tested at 8 and 24 DPA as indicated by the lane labels, and stem and leaf tissue was harvested at 18 days after treatment, if any, at 16 days. No induction of F285 expression was found in the leaves treated with SA or ETH or in seedling stems treated with ETH. However, weak expression was detected in SA-treated, but not in ETH- or H₂O-treated, 8 DPA fibers from cultured ovules (the blot was exposed to film for 4 days). These data suggest that the F285 promoter is only weakly responsive to inducers of other defense genes, and this may argue for a developmental role of F285.

### Example 6 - Identification of a Possible F285 Gene Family

Figure 6 shows a Southern blot of cotton genomic DNA (prepared from leaves of 18 day old seedlings) digested with EcoR1 and Hind III restriction enzymes and probed with the F285 full-length cDNA; the banding pattern suggests 4-5 family members. In order to begin to understand the genomic and functional diversity of a possible F285 gene family, a primer from a distinctive region (6 TAG repeats) near the 3' untranslated end of the F285 sequence was used in PCR screening of the fiber cDNA library, followed by cloning of the amplified PCR products. Three colonies were chosen for insert-end sequencing, one of which was found to be different from the other two. This clone, designated TAG1, was used for Southern (Figure 6) and Northern hybridization (Figure 7). The genomic DNA blot used for F285 hybridization was stripped and reprobed with TAG1, which hybridized to a similar set of fragments with different hybridization intensities. Therefore, F285 and TAG1 are related at the DNA level.

TAG1 was expressed in greenhouse-grown fibers at 8 DPA (primary wall stage) and up-regulated in fibers at 24 DPA (secondary wall stage). Weak expression was also detected in ovules stripped of fibers (although some contaminating fiber parts cannot be excluded) and in roots and stems. Expression was not detected in leaves. Therefore, TAG1 had a broader expression pattern than F285.

### Example 7 - Identification of the Promoter of F285

Using the ³²P labeled F285 cDNA fragment as the probe, six plaques from a lambda Fix II library (custom library, Stratagene, La Jolla, CA) were isolated after three rounds of selection. The DNA was isolated from the culture of five plaques and digested with restriction enzyme XbaI. The restriction pattern revealed that there were at least four bands in the DNA from each plaques. Southern hybridization showed that only one band, 4.3 kb in size, hybridized with the probe of F285 fragment. The 4.3 kb fragment was cut out of the gel and purified by Jetsorb gel extraction kit (PGC Scientific, Gaithersburg, MD) for subcloning.

The vector, pBS, was digested with XbaI and dephosphorylated. Ligation with 4.3 Kb fragment was conducted with T4 DNA ligase at 15°C overnight in a total volume of 5 µl. The competent cells of DH-α5 were transformed and the transformants were selected on X-gal/IPTG plates..

A PCR product, using the T3 promoter sequence and a reverse primer designed from nucleotide number 159 to 178 of the F285 sequence as the primers, showed a 2.3 kb fragment, indicating it may cover the promoter, the 5' untranslated sequence, and possibly intron(s). The sequencing was carried out with an automated oligo DNA synthesizer (Beckman Instruments Inc., Fullerton, CA). More primers were designed as chromosome walking went on until the T3 promoter sequence of the vector was passed through. A reverse sequencing of the entire sequenced region was performed to ensure the right reading of the printout of the electropherogram.

## Claims

1. An isolated nucleic acid molecule from cotton plants encoding an endogenous cotton chitinase, wherein the nucleic acid molecule is expressed preferentially in fibres during secondary wall deposition and wherein the nucleic acid molecule:
a. comprises a nucleotide sequence encoding an endogenous cotton chitinase and which hybridizes to a DNA molecule having a sequence according to SEQ.ID. No. 1 under stringent conditions **characterized by** a hybridization buffer comprising 1 x SSC at a temperature of 61 °C;
b. has the nucleotide sequence of SEQ. ID. No. 1; or
c. encodes the protein or polypeptide having the amino acid sequence of SEQ.ID.No.2.

2. The isolated nucleic acid molecule according to claim 1, wherein the fibre is cotton fibre.

3. The isolated nucleic acid molecule according to claim 2, wherein the nucleic acid molecule is expressed in cotton fibres beginning at 14 to 17 days post anthesis (DPA).

4. The isolated nucleic acid molecule according to claim 3, wherein the nucleic acid molecule is expressed in cotton fibres through to the termination of secondary wall deposition.

5. The isolated nucleic acid molecule according to claim 3, wherein the nucleic acid molecule is expressed in cotton fibres up to 31 DPA.

6. A polypeptide encoded by the nucleic acid molecule according to any one of claims 1 to 5.

7. The polypeptide according to claim 6, wherein the polypeptide comprises an amino acid sequence of SEQ. ID. No. 2.

8. A DNA construct comprising a promoter operably linked 5' to a nucleic acid molecule according to claims 1 to 5 and a 3' regulatory region operably linked to said nucleic acid molecule wherein said promoter is foreign to said nucleic acid molecule.

9. A plant cell comprising a DNA construct according to claim 8.

10. A transgenic plant cell comprising a transgene, said transgene comprising a DNA construct according to claim 8.

11. A plant cell according to claim 9 or 10, wherein the plant is selected from the group consisting of cotton, corn, sugar cane, rice, flax, hemp, ramie, jute; kenaf, kapok, coir, bamboo, spanish moss, abaca, and Agave spp.

12. A plant comprising in its cells, a DNA construct according to claim 8.

13. A transgenic plant comprising in its cells a transgene, said transgene comprising a DNA construct according to claim 8.

14. A plant according to claim 12 or 13, wherein the plant is selected from the group consisting of cotton, corn, sugar cane, rice, flax, hemp, ramie, jute, kenaf, kapok, coir, bamboo, spanish moss, abaca, and Agave spp.

15. A plant seed comprising a DNA construct according to claim 8.

16. A transgenic plant seed comprising a transgene, said transgene comprising a DNA construct according to claim 8.

17. A plant seed according to claim 15 or 16, wherein the plant is selected from the group consisting of cotton, corn, sugar cane, rice, flax, hemp, ramie, jute, kenaf, kapok, coir, bamboo, spanish moss, abaca, and Agave spp.

18. A method of regulating the cellulose content of a plant fibre comprising the step of transforming a plant with a DNA construct according to claim 8.

19. A method of providing a plant having resistance to insects and fungi comprising the step of transforming a plant with a DNA construct according to claim 8.

## Patentansprüche

1. Isoliertes Nukleinsäuremolekül von Baumwollpflanzen, das für eine endogene Baumwollchitinase kodiert, wobei das Nukleinsäuremolekül bevorzugt bei der Sekundärwandbildung in Fasern exprimiert wird und wobei das Nukleinsäuremolekül:
a. eine Nukleotidsequenz umfasst, die für eine endogene Baumwollchitinase kodiert und die unter stringenten Bedingungen **gekennzeichnet durch** einen Hybridisierungspuffer, der 1 x SSC umfasst, bei einer Temperatur von 61 °C mit einem DNA-Molekül hybridisiert, das eine Sequenz gemäß SEQ.ID. No. 1 aufweist,
b. die Nukleotidsequenz von SEQ.ID No. 1 aufweist oder
c. für das Protein oder Polypeptid mit der Aminosäuresequenz von SEQ.ID No. 2 kodiert.

2. Isoliertes Nukleinsäuremolekül nach Anspruch 1, wobei die Faser eine Baumwollfaser ist.

3. Isoliertes Nukleinsäuremolekül nach Anspruch 2, wobei das Nukleinsäuremolekül in Baumwollfasern beginnend am Tag 14 bis 17 nach einer Anthese exprimiert wird.

4. Isoliertes Nukleinsäuremolekül nach Anspruch 3, wobei das Nukleinsäuremolekül in Baumwollfasern bis zur Beendigung der Sekundärwandbildung exprimiert wird.

5. Isoliertes Nukleinsäuremolekül nach Anspruch 3, wobei das Nukleinsäuremolekül in Baumwollfasern bis zu 31 Tagen nach der Anthese exprimiert wird.

6. Polypeptid, für das das Nukleinsäuremolekül nach einem der Ansprüche 1 bis 5 kodiert.

7. Polypeptid nach Anspruch 6, wobei das Polypeptid eine Aminosäuresequenz von SEQ.ID No. 2 umfasst.

8. DNA-Konstrukt umfassend einen Promotor, der operativ 5' mit einem Nukleinsäuremolekül nach einem der Ansprüche 1 bis 5 verknüpft ist, und eine regulatorische Region 3', die operativ mit dem Nukleinsäuremolekül verknüpft ist, wobei der Promotor dem Nukleinsäuremolekül fremd ist.

9. Pflanzenzelle, die ein DNA-Konstrukt nach Anspruch 8 umfasst.

10. Transgene Pflanze, umfassend ein Transgen, wobei das Transgen ein DNA-Konstrukt nach Anspruch 8 umfasst.

11. Pflanzenzelle nach Anspruch 9 oder 10, wobei die Pflanze ausgewählt ist aus der Gruppe bestehend aus Baumwolle, Mais, Zuckerrohr, Reis, Flachs, Hanf, Ramie, Jute, Kenaf, Kapok, Kokos, Bambus, Louisianamoos, Abaca und Agave spp.

12. Pflanze, die in ihren Zellen ein DNA-Konstrukt nach Anspruch 8 umfasst.

13. Transgene Pflanzenzelle, die in ihren Zellen ein Transgen umfasst, wobei das Transgen ein DNA-Konstrukt nach Anspruch 8 umfasst.

14. Pflanze nach Anspruch 12 oder 13, wobei die Pflanze ausgewählt ist aus der Gruppe bestehend aus Baumwolle, Mais, Zuckerrohr, Reis, Flachs, Hanf, Ramie, Jute, Kenaf, Kapok, Kokos, Bambus, Louisianamoos, Abaca und Agave spp.

15. Pflanzensamen umfassend ein DNA-Konstrukt nach Anspruch 8.

16. Transgener Pflanzensamen, umfassend ein Transgen, wobei das Transgen ein DNA-Konstrukt nach Anspruch 8 umfasst.

17. Pflanzensamen nach Anspruch 15 oder 16, wobei die Pflanze ausgewählt ist aus der Gruppe bestehend aus Baumwolle, Mais, Zuckerrohr, Reis, Flachs, Hanf, Ramie, Jute, Kenaf, Kapok, Kokos, Bambus, Louisianamoos, Abaca und Agave spp.

18. Verfahren zum Regulieren des Cellulosegehalts einer Pflanzenfaser, umfassend den Schritt zum Transformieren einer Pflanze mit einem DNA-Konstrukt nach Anspruch 8.

19. Verfahren zum Bereitstellen einer Pflanze mit Resistenz gegen Insekten und Pilze, umfassend den Schritt zum Transformieren einer Pflanze mit einem DNA-Konstrukt nach Anspruch 8.

## Revendications

1. Molécule d'acide nucléique isolée à partir de plants de coton codant pour une chitinase endogène du coton, dans laquelle la molécule d'acide nucléique est exprimée préférentiellement dans des fibres pendant un dépôt secondaire sur les parois et dans laquelle la molécule d'acide nucléique :
a. comprend une séquence nucléotidique codant pour une chitinase endogène du coton et qui s'hybride avec une molécule d'ADN ayant une séquence selon SEQ ID No : 1 dans des conditions stringentes **caractérisées par** un tampon d'hybridation comprenant du 1 x SSC à une température de 61°C ;
b. a la séquence nucléotidique de SEQ ID No : 1 ; ou
c. code pour la protéine ou le polypeptide ayant la séquence d'acides aminés de SEQ ID No : 2.

2. Molécule d'acide nucléique isolée selon la revendication 1, dans laquelle la fibre est une fibre de coton.

3. Molécule d'acide nucléique isolée selon la revendication 2, dans laquelle la molécule d'acide nucléique est exprimée dans des fibres de coton avec un commencement à 14 à 17 jours post-anthèse (DPA).

4. Molécule d'acide nucléique isolée selon la revendication 3, dans laquelle la molécule d'acide nucléique est exprimée dans des fibres de coton jusqu'à la fin du dépôt secondaire sur les parois.

5. Molécule d'acide nucléique isolée selon la revendication 3, dans laquelle la molécule d'acide nucléique est exprimée dans des fibres de coton jusqu'à 31 DPA.

6. Polypeptide codé par la molécule d'acide nucléique selon l'une quelconque des revendications 1 à 5.

7. Polypeptide selon la revendication 6, dans lequel le polypeptide comprend une séquence d'acides aminés de SEQ ID No : 2.

8. Construction d'ADN comprenant un promoteur opérationnellement lié en 5' à une molécule d'acide nucléique selon les revendications 1 à 5 et une région de régulation 3' opérationnellement liée à ladite molécule d'acide nucléique, dans laquelle ledit promoteur est étranger à ladite molécule d'acide nucléique.

9. Cellule de plante comprenant une construction d'ADN selon la revendication 8.

10. Cellule de plante transgénique comprenant un transgène, ledit transgène comprenant une construction d'ADN selon la revendication 8.

11. Cellule de plante selon la revendication 9 ou 10, dans laquelle la plante est choisie parmi le groupe consistant en le coton, le maïs, la canne à sucre, le riz, le lin, le chanvre, la ramie, le jute ; le kenaf, le kapok, la fibre de coco, le bambou, la mousse espagnole, l'abaca, et *Agave spp.*

12. Plante comprenant dans ses cellules une construction d'ADN selon la revendication 8.

13. Plante transgénique comprenant dans ses cellules un transgène, ledit transgène comprenant une construction d'ADN selon la revendication 8.

14. Plante selon la revendication 12 ou 13, dans laquelle la plante est choisie parmi le groupe consistant en le coton, le maïs, la canne à sucre, le riz, le lin, le chanvre, la ramie, le jute, le kenaf, le kapok, la fibre de coco, le bambou, la mousse espagnole, l'abaca, et *Agave spp*.

15. Graine de plante comprenant une construction d'ADN selon la revendication 8.

16. Graine de plante transgénique comprenant un transgène, ledit transgène comprenant une construction d'ADN selon la revendication 8.

17. Graine de plante selon la revendication 15 ou 16, dans laquelle la plante est choisie parmi le groupe consistant en le coton, le maïs, la canne à sucre, le riz, le lin, le chanvre, la ramie, le jute, le kenaf, le kapok, la fibre de coco, le bambou, la mousse espagnole, l'abaca, et *Agave spp.*

18. Procédé de régulation de la teneur en cellulose d'une fibre de plante, comprenant l'étape de transformation d'une plante avec une construction d'ADN selon la revendication 8.

19. Procédé d'obtention d'une plante présentant une résistance aux insectes et aux champignons comprenant l'étape de transformation d'une plante avec une construction d'ADN selon la revendication 8.


### SEQUENCE LISTING

<110> Haigler, Candace H.
   Zhang, Hong
   Wu, Chunfa
   Wan, Chun-Hua
<120> CHITINASE ENCODING DNA MOLECULE FROM COTTON EXPRESSED PREFERENTIALLY IN FIBERS DURING SECONDARY WALL DEPOSITION AND THE CORRESPONDING PROMOTER
<130> 201304/1050
<140>
   <141>
<160> 10
<170> PatentIn Ver. 2.1
<210> 1
   <211> 957
   <212> DNA
   <213> Gossypium hirsutum
<400> 1
   atggaggcca aatggctgct atgttttaca atggcagcac taatggcagt gtcaaatggc 60
   caggaatcag tgaagccatt ggtgaagata gttaaaggca agaaactttg tgataaaggg 120
   tgggaatgta aagggtggtc acagttttgt tgtaaccaaa ccatttctga ttatttccga 180
   acttatcaat ttgagaacct tttcgctaaa cgtaatacac cggtggcaca tgcggttggg 240
   ttctgggatt accattcttt cattacggcg gcggctcagt atcagcctca tggttttggt 300
   accaccggcg gtaagctgca gagcatgaag gaagtggcag cttttcttgg acatgtcggc 360
   agcaaaactt catgtggtta tggagtggca actgggggac cattggcttg gggtctatgc 420
   tacaacaagg aaatgagtcc tagcaaattg tattgtgatg attactacaa atacacctac 480
   ccttgcactc ctggagtttc ttaccatggc cgtggtgcct tgcctatcta ttggaactac 540
   aactatggag aaacaggcga cgcattgaag gtggacttat tgaaccaccc tgaatacata 600
   gaaaacaatg caaccttagc tttccaggca gcactctgga gatggatgac accggtgaag 660
   aaacaccaac cgtcggccca cgacgtgttt gtcggcagct ggaaaccgac caagaacgac 720
   acgttggcca agcgggtccc ggggtttgga gccaccatga atgtgctcta tggagatcaa 780
   gtttgtgggc gaggtgatgt tgacaccatg aacaacatca tctctcatta cctttcttac 840
   cttgacctaa tgggagttgg gagagaagag gcaggacccc atgaagtgct cacatgtgaa 900
   gaacaaaagc ctttcactgt atctccttct tctgcatcat catcatcatc atcttga    957
<210> 2
   <211> 318
   <212> PRT
   <213> Gossypium hirsutum
<400> 2
   Met Glu Ala Lys Trp Leu Leu Cys Phe Thr Met Ala Ala Leu Met Ala
   1 5 10 15
   Val Ser Asn Gly Gln Glu Ser Val Lys Pro Leu Val Lys Ile Val Lys
   20 25 30
   Gly Lys Lys Leu Cys Asp Lys Gly Trp Glu Cys Lys Gly Trp Ser Gln
   35 40 45
   Phe Cys Cys Asn Gln Thr Ile Ser Asp Tyr Phe Arg Thr Tyr Gln Phe
   50 55 60
   Glu Asn Leu Phe Ala Lys Arg Asn Thr Pro Val Ala His Ala Val Gly
   65 70 75 80
   Phe Trp Asp Tyr His Ser Phe Ile Thr Ala Ala Ala Gln Tyr Gln Pro
   85 90 95
   His Gly Phe Gly Thr Thr Gly Gly Lys Leu Gln Ser Met Lys Glu Val
   100 105 110
   Ala Ala Phe Leu Gly His Val Gly Ser Lys Thr Ser Cys Gly Tyr Gly
   115 120 125
   Val Ala Thr Gly Gly Pro Leu Ala Trp Gly Leu Cys Tyr Asn Lys Glu
   130 135 140
   Met Ser Pro Ser Lys Leu Tyr Cys Asp Asp Tyr Tyr Lys Tyr Thr Tyr
   145 150 155 160
   Pro Cys Thr Pro Gly Val Ser Tyr His Gly Arg Gly Ala Leu Pro Ile
   165 170 175
   Tyr Trp Asn Tyr Asn Tyr Gly Glu Thr Gly Asp Ala Leu Lys Val Asp
   180 185 190
   Leu Leu Asn His Pro Glu Tyr Ile Glu Asn Asn Ala Thr Leu Ala Phe
   195 200 205
   Gln Ala Ala Leu Trp Arg Trp Met Thr Pro Val Lys Lys His Gln Pro
   210 215 220
   Ser Ala His Asp Val Phe Val Gly Ser Trp Lys Pro Thr Lys Asn Asp
   225 230 235 240
   Thr Leu Ala Lys Arg Val Pro Gly Phe Gly Ala Thr Met Asn Val Leu
   245 250 255
   Tyr Gly Asp Gln Val Cys Gly Arg Gly Asp Val Asp Thr Met Asn Asn
   260 265 270
   Ile Ile Ser His Tyr Leu Ser Tyr Leu Asp Leu Met Gly Val Gly Arg
   275 280 285
   Glu Glu Ala Gly Pro His Glu Val Leu Thr Cys Glu Glu Gln Lys Pro
   290 295 300
   Phe Thr Val Ser Pro Ser Ser Ala Ser Ser Ser Ser Ser Ser
   305 310 315
<210> 3
   <211> 1022
   <212> DNA
   <213> Unknown Organism
<220>
   <223> Description of Unknown Organism: Promoter sequence
<400> 3
   atgaaacatc ttcgtactca tatctgaaac tccagcttct tgatcctcaa tgataattaa 60
   atcctcacta tcactcgcat tcacctcgag cagcttcgca aattgaaatg tttccttagc 120
   ttccttcact atacttgcga ttcccaatga cagagtcagt aagggaacca ttaacaatac 180
   gatcatccgt tctttgcttc ttcaccagac accacacctt tagatatgat tggttttcta 240
   cctctacgtt tttgcttctt tttttttttt aaccaaagtc atcacttttt cttcaatctc 300
   tgccaatgat ctcaccttct tcttcacacc atcaacagta attatcgtcg aatagggatt 360
   atgcactaca gtgttttgca ttgctactcc catggagtcc tccaccctaa ctctacttgt 420
   aggactccta actaacgaat tgcaaagggt ttcgcgaccc gaattgttcc ctttctgaca 480
   acccaagtcc acttaggttc aatcatattt aaattcgtat caacaatctc ctcagccgac 540
   caattcacag ctttcaaatc tgctccgcaa cccaccattt gatcgtgacc aaagtgtgaa 600
   cttgccttca acaaatcagg cccagagcct cgttctaatc atttctcgag gcaatagcaa 660
   tagttgggtc taagttctct gctaattcct ttgatttcct agaacctctc gagctgccat 720
   attgggtttt tcactaccca cctcttcatt aaatgtatct tcaacctctc aactcctttc 780
   accaccagac gaatcttctt tagcaaaatc aaaatgacct tatgaaaatt tagcacgtcc 840
   acctccagat tcaaaggctg tgaatcccca acttcggaaa ttgttcatct ccacattcaa 900
   gaataatgag ttcctcaatt tgttttaact gattagccga tattaagcga gttagactcc 960
   atggaaataa aatcacccta ataaatagca acgcttttga acgtctctag gttccaagcg 1020
   tg 1022
<210> 4
   <211> 59
   <212> PRT
   <213> Arabidopsis thaliana
<220>
   <221> SIMILAR
   <222> (18)
   <223> X at position 18 is an amino acid that is weakly or strongly similar to I
<220>
   <221> SIMILAR
   <222> (26)
   <223> X at position 26 is an amino acid that is weakly or strongly similar to E
<220>
   <221> SIMILAR
   <222> (29)
   <223> X at position 29 is an amino acid that is weakly or strongly similar to D
<220>
   <221> SIMILAR
   <222> (37)
   <223> X at position 37 is an amino acid that is weakly or strongly similar to N
<220>
   <221> SIMILAR
   <222> (42)
   <223> X at position 42 is an amino acid that is weakly or strongly similar to I
<220>
   <221> SIMILAR
   <222> (54)
   <223> X at position 54 is an amino acid that is weakly or strongly similar to L
<400> 4
   Thr Tyr Pro Cys Thr Pro Gly Val Ser Tyr His Gly Arg Gly Ala Leu
   1               5                   10                  15
   Pro Xaa Tyr Trp Asn Tyr Asn Tyr Gly Xaa Thr Gly Xaa Ala Leu Lys
               20                  25                  30
   Val Asp Leu Leu Xaa His Pro Glu Tyr Xaa Glu Asn Asn Ala Thr Leu
           35                  40                  45
   Ala Phe Gln Ala Ala Xaa Trp Arg Trp Met Thr
       50                  55
<210> 5
   <211> 59
   <212> PRT
   <213> Arabis microphylla
<220>
   <221> SIMILAR
   <222> (2)
   <223> X at position 2 is an amino acid that is weakly or strongly similar to Y
<220>
   <221> SIMILAR
   <222> (5)
   <223> X at position 5 is an amino acid that is weakly or strongly similar to T
<220>
   <221> SIMILAR
   <222> (11)
   <223> X at position 11 is an amino acid that is weakly or strongly similar to H
<220>
   <221> SIMILAR
   <222> (15)
   <223> X at position 15 is an amino acid that is weakly or strongly similar to A
<220>
   <221> SIMILAR
   <222> (16)
   <223> X at position 16 is an amino acid that is weakly or strongly similar to L
<220>
   <221> SIMILAR
   <222> (18)
   <223> X at position 18 is an amino acid that is weakly or strongly similar to I
<220>
   <221> SIMILAR
   <222> (31)
   <223> X at position 31 is an amino acid that is weakly or strongly similar to L
<220>
   <221> SIMILAR
   <222> (38)
   <223> X at position 38 is an amino acid that is weakly or strongly similar to H
<220>
   <221> SIMILAR
   <222> (40)
   <223> X at position 40 is an amino acid that is weakly or strongly similar to E
<220>
   <221> SIMILAR
   <222> (42)
   <223> X at position 42 is an amino acid that is weakly or strongly similar to I
<220>
   <221> SIMILAR
   <222> (45)
   <223> X at position 45 is an amino acid that is weakly or strongly similar to N
<220>
   <221> SIMILAR
   <222> (47)
   <223> X at position 47 is an amino acid that is weakly or strongly similar to T
<220>
   <221> SIMILAR
   <222> (48)
   <223> X at position 48 is an amino acid that is weakly or strongly similar to L
<220>
   <221> SIMILAR
   <222> (51)
   <223> X at position 51 is an amino acid that is weakly or strongly similar to Q
<220>
   <221> SIMILAR
   <222> (54)
   <223> X at position 54 is an amino acid that is weakly or strongly similar to L
<400> 5
   Thr Xaa Pro Cys Xaa Pro Gly Lys Arg Tyr Xaa Gly Arg Gly Xaa Xaa
   1               5                   10                  15
   Gln Xaa Ser Trp Asn Tyr Asn Tyr Gly Leu Cys Gly Arg Ala Xaa Gly
               20                  25                  30
   Val Asp Leu Leu Asn Xaa Pro Xaa Leu Xaa Ala Asn Xaa Ala Xaa Xaa
           35                  40                  45
   Ala Phe Xaa Ala Ala Xaa Trp Phe Trp Met Thr
       50                  55
<210> 6
   <211> 59
   <212> PRT
   <213> Gossypium hirsutum
<220>
   <221> SIMILAR
   <222> (1)
   <223> X at position 1 is an amino acid that is weakly or strongly similar to T
<220>
   <221> SIMILAR
   <222> (5)
   <223> X at position 5 is an amino acid that is weakly or strongly similar to T
<220>
   <221> SIMILAR
   <222> (9)
   <223> X at position 9 is an amino acid that is weakly or strongly similar to S
<220>
   <221> SIMILAR
   <222> (11)
   <223> X at position 11 is an amino acid that is weakly or strongly similar to H
<220>
   <221> SIMILAR
   <222> (15)
   <223> X at position 15 is an amino acid that is weakly or strongly similar to A
<220>
   <221> SIMILAR
   <222> (16)
   <223> X at position 16 is an amino acid that is weakly or strongly similar to L
<220>
   <221> SIMILAR
   <222> (18)
   <223> X at position 18 is an amino acid that is weakly or strongly similar to I
<220>
   <221> SIMILAR
   <222> (26)
   <223> X at position 26 is an amino acid that is weakly or strongly similar to E
<220>
   <221> SIMILAR
   <222> (31)
   <223> X at position 31 is an amino acid that is weakly or strongly similar to L
<220>
   <221> SIMILAR
   <222> (32)
   <223> X at position 32 is an amino acid that is weakly or strongly similar to K
<220>
   <221> SIMILAR
   <222> (38)
   <223> X at position 38 is an amino acid that is weakly or strongly similar to H
<220>
   <221> SIMILAR
   <222> (40)
   <223> X at position 40 is an amino acid that is weakly or strongly similar to E
<220>
   <221> SIMILAR
   <222> (42)
   <223> X at position 42 is an amino acid that is weakly or strongly similar to I
<220>
   <221> SIMILAR
   <222> (43)
   <223> X at position 43 is an amino acid that is weakly or strongly similar to E
<220>
   <221> SIMILAR
   <222> (44)..(45)
   <223> X at positions 44 and 45 is an amino acid that is weakly or strongly similar to N
<220>
   <221> SIMILAR
   <222> (46)
   <223> X at position 46 is an amino acid that is weakly or strongly similar to A
<220>
   <221> SIMILAR
   <222> (48)
   <223> X at position 48 is an amino acid that is weakly or strongly similar to L
<220>
   <221> SIMILAR
   <222> (49)
   <223> X at position 49 is an amino acid that is weakly or strongly similar to A
<220>
   <221> SIMILAR
   <222> (51)
   <223> X at position 51 is an amino acid that is weakly or strongly similar to Q
<220>
   <221> SIMILAR
   <222> (52)
   <223> X at position 52 is an amino acid that is weakly or strongly similar to A
<220>
   <221> SIMILAR
   <222> (54)
   <223> X at position 54 is an amino acid that is weakly or strongly similar to L
<220>
<400> 6
   Xaa Tyr Pro Cys Xaa Pro Gly Lys Xaa Tyr Xaa Gly Arg Gly Xaa Xaa
    1               5                  10                  15
   Gln Xaa Ser Trp Asn Tyr Asn Tyr Gly Xaa Cys Gly Arg Ala Xaa Xaa
               20                  25                  30
   Val Asp Leu Leu Asn Xaa Pro Xaa Leu Xaa Xaa Xaa Xaa Xaa Thr Xaa
           35                  40                  45
   Xaa Phe Xaa Xaa Ala Xaa Trp Phe Trp Met Thr
       50                  55
<210> 7
   <211> 21
   <212> PRT
   <213> Artificial Sequence
<220>
   <223> Description of Artificial Sequence: Consensus sequence
<400> 7
   Gly Arg Gly Pro Ile Gln Leu Ser Xaa Trp Asn Tyr Asn Tyr Gly Pro
    1               5                  10                  15
   Ala Gly Arg Ala Ile
               20
<210> 8
   <211> 39
   <212> PRT
   <213> Artificial Sequence
<220>
   <223> Description of Artificial Sequence: Signature sequence of putatively unchanging amino acids
<400> 8
   Lys Arg Glu Val Ala Ala Phe Leu Ala Gln Thr Ser His Glu Thr Thr
    1               5                  10                  15
   Gly Gly Trp Ala Thr Ala Pro Asp Gly Ala Phe Ala Trp Gly Tyr Cys
               20                  25                  30
   Phe Lys Gln Glu Arg Gly Ala
           35
<210> 9
   <211> 39
   <212> PRT
   <213> Arabis microphylla
<400> 9
   Lys Lys Glu Ile Ala Ala Phe Phe Gly Gln Thr Ser His Glu Thr Thr
    1               5                  10                  15
   Gly Gly Trp Ala Ser Ala Pro Asp Gly Pro Phe Ser Trp Gly Tyr Cys
               20                  25                  30
   Phe Lys Gln Glu Gln Asn Pro
           35
<210> 10
   <211> 39
   <212> PRT
   <213> Arabidopsis thaliana
<400> 10
   Met Lys Glu Val Ala Ala Phe Leu Gly His Val Gly Ser Lys Thr Ser
    1               5                  10                  15
   Cys Gly Tyr Gly Val Ala Thr Gly Gly Pro Leu Ala Trp Gly Leu Cys
               20                  25                  30
   Tyr Asn Lys Glu Met Ser Pro
           35